# EUROPEAN PATENT APPLICATION

(11) **EP 4 810 030 A1**
(43) Date of publication of application: **23.09.2026**
(21) Application number: 24890769.3
(22) Date of filing: 14.11.2024
(51) Int. Cl.: A61K 9/51, A61K 47/26, C12N 15/86, A61P 31/16

(54) **PREPARATION METHOD FOR AND USE OF LIPID NANOPARTICLES**

(30) Priority: 14.11.2023 CN 202311517076
(71) Applicant: Shanghai Regenelead Therapies Co., Ltd., Shanghai 201206 (CN)
(72) Inventor: LIU, Chongyi, Shanghai 201206 (CN); TANG, Xiaojiao, Shanghai 201206 (CN); WANG, Qin, Shanghai 201206 (CN); KUANG, Jingwen, Shanghai 201206 (CN); JIANG, Jun, Shanghai 201206 (CN); NING, Wei, Shanghai 201206 (CN); LIAO, Cheng, Shanghai 201206 (CN)
(74) Representative: Potter Clarkson
(86) International application number: PCT/CN2024/132052
(87) International publication number: WO 2025/103414

(57) **Abstract**

The present invention relates to a preparation method for and the use of lipid nanoparticles. Specifically, provided is a freeze-dried composition, comprising lipid nanoparticles and sodium chloride, wherein the lipid nanoparticles comprise at least one cationic lipid, and the weight ratio of sodium chloride to the cationic lipid is 1:10-10:1.

## Description

### FIELD OF THE INVENTION

The present disclosure belongs to the field of medicine, and relates to a preparation method for and use of lipid nanoparticles.

### BACKGROUND OF THE INVENTION

Nucleic acid-based drugs, such as messenger RNAs (mRNA), antisense oligonucleotides, small interfering RNAs (siRNA), plasmids, etc., have a wide application prospect. At present, the delivery carriers for nucleic acid drugs can be divided into two categories: viral carriers and non-viral carriers. Among them, lipid nanoparticle-mediated delivery for nucleic acid drugs is the main means of the non-viral delivery carriers.

In gene therapy and vaccine applications, lipid nanoparticles have proven to be excellent delivery carriers for nucleic acids. Lipid nanoparticles formed from cationic lipids and other helper lipids such as cholesterol, phospholipids and PEGylated lipids can encapsulate nucleic acids, protecting them from degradation and promoting cellular uptake, thus reducing immune responses. In addition, intracellular delivery of nucleic acids via lipid nanoparticles has other advantages, such as good targeting, fewer side effect, good stability, and high transfection efficiency. However, commercially available mRNA vaccines require cryopreservation, resulting in high transportation and storage costs, greatly limiting their application range. Therefore, the exploration of other types of formulations and preparation methods such as lyophilization makes it possible to significantly increase the preservation temperature of lipid nanoparticles. However, the preparation and lyophilization processes can affect the particle sizes of lipid nanoparticles. The average particle size of lipid nanoparticles is an important determinant of their properties, and the particle size influences drug metabolism, *in vivo* distribution, efficacy, and safety. Reports have indicated that, in NPH animal models, LNPs with a particle size of 150 nm can still trigger strong immune responses (Mol Ther. 2022 May 4; 30 (5): 1941-1951). Therefore, it is an important quality standard for lipid nanoparticles to maintain the particle size of lyophilized and reconstituted nanoparticles.

### SUMMARY OF THE INVENTION

The present disclosure provides a lyophilized composition comprising lipid nanoparticles and sodium chloride, wherein the lipid nanoparticles comprise at least one cationic lipid, and the weight ratio of sodium chloride to the cationic lipid ranges from 1:10 to 10:1.

In some embodiments, the weight ratio of sodium chloride to the cationic lipid in the lyophilized composition of the present disclosure ranges from 1:10 to 5:1.

In some embodiments, the weight ratio of sodium chloride to the cationic lipid in the lyophilized composition of the present disclosure ranges from 1:5 to 10:1.

In some embodiments, the weight ratio of sodium chloride to the cationic lipid in the lyophilized composition of the present disclosure ranges from 1:5 to 5:1.

In some embodiments, the weight ratio of sodium chloride to the cationic lipid in the lyophilized composition of the present disclosure ranges from 1:3 to 5:1.

In some embodiments, the weight ratio of sodium chloride to the cationic lipid in the lyophilized composition of the present disclosure ranges from 1:2 to 5:1.

In some embodiments, the weight ratio of sodium chloride to the cationic lipid in the lyophilized composition of the present disclosure ranges from 1:2 to 4:1.

In some embodiments, the weight ratio of sodium chloride to the cationic lipid in the lyophilized composition of the present disclosure ranges from 1:5 to 3:1.

In some embodiments, the weight ratio of sodium chloride to the cationic lipid in the lyophilized composition of the present disclosure ranges from 1:3 to 3:1.

In some embodiments, the weight ratio of sodium chloride to the cationic lipid in the lyophilized composition of the present disclosure ranges from 1:3 to 2:1.

In some embodiments, the weight ratio of sodium chloride to the cationic lipid in the lyophilized composition of the present disclosure ranges from 1:2 to 2:1.

In some embodiments, the cationic lipid in the present disclosure can be selected from the group consisting of N,N-dioleyl-N,N-dimethylammonium chloride (DODAC), N,N-distearyl-N,N-dimethylammonium bromide (DDAB), 1,2-dioleoyltrimethylammonium propane chloride (DOTAP)(also known as N-(2,3-dioleoyloxy)propyl)-N,N,N-trimethylammonium chloride and 1,2-dioleoyloxy-3-trimethylaminopropane chloride salt), N-(1-(2,3-dioleoyloxy)propyl)-N,N,N-trimethylammonium chloride (DOTMA), N,N-dimethyl-2,3-dioleoyloxy)propylamine (DODMA), 1,2-dilinoleyloxy-N,N-dimethylaminopropane (DLinDMA), 1,2-dilinolenyloxy-N,N-dimethylaminopropane (DLenDMA), 1,2-di-γ-linolenyloxy-N,N- dimethylaminopropane (γ-DLenDMA), 1,2-dilinoleylcarbamoyloxy-3-dimethylaminopropane (DLin-C-DAP), 1,2-dilinoleyloxy-3-(dimethylamino)acetoxypropane (DLin-DAC), 1,2-dilinoleyloxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazinyl)propane (DLin-MPZ) or 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cy clopenta[d][1,3]dioxol-5-amine, (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl) piperazin-1-yl)ethylazanediyl)didodecan-2-ol (C12-200), 2,2-dilinoleyl-4-(2-dimethylaminoethyl)-[1,3]-dioxolane (DLin-K-C2-DMA), 2,2-dilinoleyl-4-dimethylaminoethyl-[1,3]-dioxolane (DLin-K-DMA), (6Z,9Z,28Z,31Z)-heptatriaconta-6,9,28,31-tetraen-19-yl 4-(dimethylamino)butanoate (DLin-M-C3-DMA), 3-((6Z,9Z,28Z,31Z)-heptatriaconta-6,9,28,31-tetraen-19-yloxy)-N,N-dimethylpropan-1-amine (MC3 Ether), 4-((6Z,9Z,28Z,31Z)-heptatriaconta-6,9,28,31-tetraen-19-yloxy)-N,N-dimethylbutan-1-amine (MC4 Ether), ((4-hydroxybutyl)azanediyl)bis(hexan-6,1-diyl)bis(2-hexyldecanoate) (ALC-0315), 1-octylnonyl 8-[(2-hydroxyethyl)[6-oxo-6-(undecyloxy)hexyl]amino]octanoate (SM-102), a compound of formula I, or any combination of the above-mentioned cationic lipids.

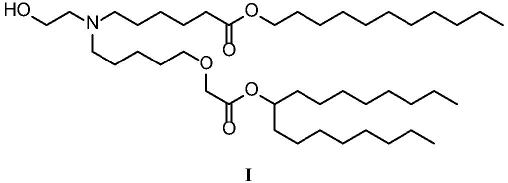

In some embodiments, the cationic lipid in the present disclosure is selected from the group consisting of tertiary amine lipids.

In some embodiments, the cationic lipid in the present disclosure comprises a tertiary amine, an ester linker, and a branched tail.

In some embodiments, the cationic lipid in the present disclosure comprises a tertiary amine, an ester linker, and at least three branched tails.

In some embodiments, the cationic lipid in the present disclosure comprises a tertiary amine, an ester linker, and three branched tails.

In some embodiments, the cationic lipid in the present disclosure comprises a tertiary amine, an ester linker, and at least four branched tails.

In some embodiments, the branched tail in the present disclosure can be a saturated or unsaturated hydrophobic tail containing 6-20 carbon atoms.

In some embodiments, the branched tail in the present disclosure can be a saturated hydrophobic tail containing 6-20 carbon atoms.

In some embodiments, the cationic lipid in the present disclosure comprises a free hydroxyl end.

In some embodiments, the cationic lipid in the present disclosure is a compound of formula A, 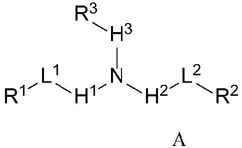
wherein, L¹ and L² are each independently selected from the group consisting of -C(O)O-, -OC(O)-, -C(O)-, -OC(O)O-, -O-, -S(O)ₓ-, -S-S-, -C(O)S-, -SC(O)-, -NR^{a}C(O)-, -C(O)NR^{a}-, -NR^{a}C(O)NR^{a}-, -NR^{a}C(O)O-, -OC(O)NR^{a}- and a bond, and R^{a} is selected from the group consisting of hydrogen, C₁₋₆ alkyl, and C₂₋₆ alkenyl;
H¹ and H² are each independently selected from the group consisting of C₁₋₁₂ heteroalkylene, C₁₋₁₂ alkylene, and C₂₋₁₂ alkenylene;
H³ is selected from the group consisting of C₁₋₂₄ alkylene, C₂₋₂₄ alkenylene, C₃₋₈ cycloalkylene and C₃₋₈ cycloalkenylene;
R¹ and R² are each independently selected from the group consisting of C₁₋₂₄ alkyl and C₂₋₂₄ alkenyl;
R³ is selected from the group consisting of hydrogen, -CN, -C(O)OR⁴, -OC(O)R⁴, -OR⁵ and -NR⁵C(O)R⁴, R⁴ is selected from the group consisting of C₁₋₆ alkyl and C₂₋₆ alkenyl, and R⁵ is selected from the group consisting of hydrogen, C₁₋₆ alkyl and C₂₋₆ alkenyl;
x is selected from the group consisting of 0, 1 and 2.

In some embodiments, the cationic lipid in the present disclosure is a compound of formula A, 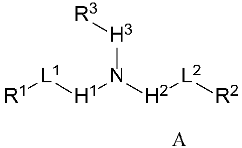
wherein, L¹ and L² are each independently selected from the group consisting of -C(O)O-, -OC(O)-, -C(O)-, -OC(O)O-, and -O-;
H¹ and H² are each independently selected from the group consisting of C₁₋₁₂ heteroalkylene, C₁₋₁₂ alkylene, and C₂₋₁₂ alkenylene;
H³ is selected from the group consisting of C₁₋₂₄ alkylene, C₁₋₂₄ heteroalkylene, and C₂₋₂₄ alkenylene;
R¹ and R² are each independently selected from the group consisting of C₁₋₂₄ alkyl and C₂₋₂₄ alkenyl;
R³ is selected from the group consisting of hydrogen, -CN, -C(O)OR⁴, -OC(O)R⁴, -OR⁵ and -NR⁵C(O)R⁴, R⁴ is selected from the group consisting of C₁₋₆ alkyl and C₂₋₆ alkenyl, and R⁵ is selected from the group consisting of hydrogen, C₁₋₆ alkyl and C₂₋₆ alkenyl.

In some embodiments, the cationic lipid in the present disclosure is a compound of formula A, 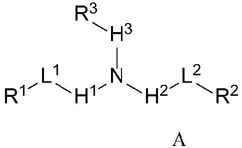
wherein, L¹ and L² are each independently selected from the group consisting of -C(O)O-, and -OC(O)-;
H¹ and H² are each independently selected from the group consisting of C₁₋₁₂ heteroalkylene, C₁₋₁₂ alkylene, and C₂₋₁₂ alkenylene;
H³ is selected from the group consisting of C₁₋₂₄ alkylene, C₁₋₂₄ heteroalkylene, and C₂₋₂₄ alkenylene;
R¹ and R² are each independently selected from the group consisting of C₁₋₂₄ alkyl and C₂₋₂₄ alkenyl;
R³ is selected from the group consisting of hydrogen, -CN, -C(O)OR⁴, -OC(O)R⁴, -OR⁵ and -NR⁵C(O)R⁴, R⁴ is selected from the group consisting of C₁₋₆ alkyl and C₂₋₆ alkenyl, and R⁵ is selected from the group consisting of hydrogen, C₁₋₆ alkyl and C₂₋₆ alkenyl.

In some embodiments, the cationic lipid in the present disclosure is a compound of formula A, 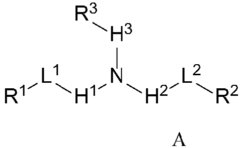
wherein, L¹ and L² are each independently selected from the group consisting of -C(O)O-, and -OC(O)-;
H¹ and H² are each independently selected from the group consisting of C₁₋₁₂ heteroalkylene, C₁₋₁₂ alkylene, and C₂₋₁₂ alkenylene;
H³ is selected from the group consisting of C₁₋₂₄ alkylene, C₁₋₂₄ heteroalkylene, and C₂₋₂₄ alkenylene;
R¹ and R² are each independently selected from the group consisting of C₁₋₂₄ alkyl and C₂₋₂₄ alkenyl;
R³ is -OR⁵, and R⁵ is hydrogen.

In some embodiments, at least one of H¹ and H² in the compound of formula A or the salt thereof is heteroalkylene, and the heteroalkylene contains at least one heteroatom selected from the group consisting of O, N, and S.

In some embodiments, H¹ in the compound of formula A or the salt thereof is selected from the group consisting of C₁₋₁₂ heteroalkylene, preferably C₂₋₉ heteroalkylene. In some embodiments, the heteroalkylene is one containing at least one oxygen atom. In some embodiments, the heteroalkylene is one containing two oxygen atoms. In some embodiments, the heteroalkylene is one containing at least one nitrogen atom. In some embodiments, the heteroalkylene is one containing at least one oxygen atom.

In some embodiments, at least one of H¹ and H² in the compound of formula A or the salt thereof is C₁₋₉ alkylene or C₂₋₁₀ alkenylene.

In some embodiments, H³ in the compound of formula A or the salt thereof is selected from the group consisting of C₂₋₂₄ alkenylene; or, H³ is selected from the group consisting of C₁₋₂₄ alkylene. In other embodiments, H³ is selected from the group consisting of C₁₋₂₄ heteroalkylene.

In some embodiments, H³ in the compound of formula A or the salt thereof is selected from C₁₋₈ alkylene. In some embodiments, H³ in the compound of formula A or the salt thereof is selected from C₂₋₆ alkylene.

In some embodiments, L¹ and L² in the compound of formula A or the salt thereof are -C(O)O-.

In some embodiments, L¹ and L² in the compound of formula A or the salt thereof are -OC(O)-.

In some embodiments, R¹ and R² in the compound of formula A or the salt thereof are each independently selected from the group consisting of C₂₋₂₄ alkyl (including but not limited to C₂ alkyl, C₃ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, and C₂₁alkyl). In other embodiments, R¹ and R² in the compound of formula A or the salt thereof are each independently selected from the group consisting of C₄₋₁₈ alkyl.

In some embodiments, R¹ and R² in the compound of formula A or the salt thereof are each independently selected from the group consisting of branched C₄₋₁₈ alkyl.

In some embodiments, R¹ and R² in the compound of formula A or the salt thereof are each independently selected from the group consisting of linear C₄₋₁₈ alkyl.

In some embodiments, in the compound of formula A or the salt thereof, R¹ is selected from the group consisting of linear C₄₋₁₈ alkyl, and R² is selected from the group consisting of branched C₄₋₁₈ alkyl.

In some embodiments, in the compound of formula A or the salt thereof, R¹ is selected from the group consisting of branched C₄₋₁₈ alkyl, and R² is selected from the group consisting of branched C₄₋₁₈ alkyl.

In some embodiments, R¹ and R² in the compound of formula A or the salt thereof are each independently selected from the group consisting of C₂₋₂₄ alkenyl (including but not limited to C₂ alkenyl, C₃ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₀ alkenyl, and C₂₁ alkenyl). In other embodiments, R¹ and R² in the compound of formula A or the salt thereof are each independently selected from the group consisting of C₄₋₁₈ alkenyl.

In some embodiments, R¹ and R² in the compound of formula A or the salt thereof are each independently selected from the group consisting of branched C₄₋₁₈ alkenyl.

In some embodiments, R¹ and R² in the compound of formula A or the salt thereof are each independently selected from the group consisting of linear C₄₋₁₈ alkenyl.

In some embodiments, in the compound of formula A or the salt thereof, R¹ is selected from the group consisting of linear C₄₋₁₈ alkenyl, and R² is selected from the group consisting of branched C₄₋₁₈ alkenyl.

In some embodiments, in the compound of formula A or the salt thereof, R¹ is selected from the group consisting of branched C₄₋₁₈ alkenyl, and R² is selected from the group consisting of branched C₄₋₁₈ alkenyl.

In some embodiments, the cationic lipid in the present disclosure is a compound of formula I.

In some embodiments, the cationic lipid in the present disclosure is ((4-hydroxybutyl)azanediyl)bis(hexan-6,1-diyl)bis(2-hexyldecanoate) (ALC-0315).

In some embodiments, the cationic lipid in the present disclosure is 1-octylnonyl 8-[(2-hydroxyethyl)[6-oxo-6-(undecyloxy)hexyl]amino]octanoate ( SM-102).

In some embodiments, the cationic lipid in the lyophilized composition comprises from 10 mol % to 75 mol %, including but not limited to 15 mol %, 16 mol %, 17 mol %, 18 mol %, 19 mol %, 20 mol %, 21 mol %, 22 mol %, 23 mol %, 24 mol %, 25 mol %, 26 mol %, 27 mol %, 28 mol %, 29 mol %, 30 mol %, 31 mol %, 32 mol %, 33 mol %, 34 mol %, 35 mol %, 36 mol %, 37 mol %, 38 mol %, 39 mol %, 40 mol %, 41 mol %, 42 mol %, 43 mol %, 44 mol %, 45 mol %, 46 mol %, 47 mol %, 48 mol %, 49 mol %, 50 mol %, 51 mol %, 52 mol %, 53 mol %, 54 mol %, 55 mol %, 56 mol %, 57 mol %, 58 mol %, 59 mol %, 60 mol %, 61 mol %, 62 mol %, 63 mol %, 64 mol %, 65 mol %, 66 mol %, 67 mol %, 68 mol %, 69 mol %, 70 mol %, 71 mol %, 72 mol %, 73 mol %, 74 mol %, 75 mol %, or a value between any two of these numbers, of total lipids present in the lipid nanoparticles.

In some embodiments, the cationic lipid in the lyophilized composition comprises from 15 mol % to 49 mol %, including but not limited to 15 mol %, 16 mol %, 17 mol %, 18 mol %, 19 mol %, 20 mol %, 21 mol %, 22 mol %, 23 mol %, 24 mol %, 25 mol %, 26 mol %, 27 mol %, 28 mol %, 29 mol %, 30 mol %, 31 mol %, 32 mol %, 33 mol %, 34 mol %, 35 mol %, 36 mol %, 37 mol %, 38 mol %, 39 mol %, 40 mol %, 41 mol %, 42 mol %, 43 mol %, 44 mol %, 45 mol %, 46 mol %, 47 mol %, 48 mol %, 49 mol %, or a value between any two of these numbers, of total lipids present in the lipid nanoparticles.

In some embodiments, the cationic lipid in the lyophilized composition comprises from 20 mol % to 70 mol % of total lipids present in the lipid nanoparticles.

In some embodiments, the cationic lipid in the lyophilized composition comprises from 30 mol % to 70 mol % of total lipids present in the lipid nanoparticles.

In some embodiments, the cationic lipid in the lyophilized composition comprises from 20 mol % to 60 mol % of total lipids present in the lipid nanoparticles.

In some embodiments, the cationic lipid in the lyophilized composition comprises from 30 mol % to 60 mol % of total lipids present in the lipid nanoparticles.

In some embodiments, the cationic lipid in the lyophilized composition comprises from 42 mol % to 49 mol % of total lipids present in the lipid nanoparticles.

In some embodiments, the cationic lipid in the lyophilized composition comprises 45 mol % of total lipids present in the lipid nanoparticles.

In some embodiments, the cationic lipid in the lyophilized composition comprises 46 mol % of total lipids present in the lipid nanoparticles.

In some embodiments, the cationic lipid in the lyophilized composition comprises 47 mol % of total lipids present in the lipid nanoparticles.

In some embodiments, the cationic lipid in the lyophilized composition comprises 48 mol % of total lipids present in the lipid nanoparticles.

In some embodiments, the cationic lipid in the lyophilized composition comprises 49 mol % of total lipids present in the lipid nanoparticles.

In some embodiments, provided is the lyophilized composition, wherein the cationic lipid comprises from 5 mol % to 40 mol %, including but not limited to 5 mol %, 6 mol %, 7 mol %, 8 mol %, 9 mol %, 10 mol %, 11 mol %, 12 mol %, 13 mol %, 14 mol %, 15 mol %, 16 mol %, 17 mol %, 18 mol %, 19 mol %, 20 mol %, 21 mol %, 22 mol %, 23 mol %, 24 mol %, 25 mol %, 26 mol %, 27 mol %, 28 mol %, 29 mol %, 30 mol %, 31 mol %, 32 mol %, 33 mol %, 34 mol %, 35 mol %, 36 mol %, 37 mol %, 38 mol %, 39 mol %, 40 mol %, or a value between any two of these numbers, of total lipids present in the lipid nanoparticles.

In some embodiments, provided is the lyophilized composition, wherein the cationic lipid comprises from 40 mol % to 70 mol %, including but not limited to 40 mol %, 41 mol %, 42 mol %, 43 mol %, 44 mol %, 45 mol %, 46 mol %, 47 mol %, 48 mol %, 49 mol %, 50 mol %, 51 mol %, 52 mol %, 53 mol %, 54 mol %, 55 mol %, 56 mol %, 57 mol %, 58 mol %, 59 mol %, 60 mol %, 61 mol %, 62 mol %, 63 mol %, 64 mol %, 65 mol %, 66 mol %, 67 mol %, 68 mol %, 69 mol %, 70 mol %, 71 mol %, 72 mol %, 73 mol %, 74 mol %, 75 mol %, or a value between any two of these numbers, of total lipids present in the lipid nanoparticles.

In some embodiments, the lipid nanoparticles in the lyophilized composition of the present disclosure comprise at least one non-cationic lipid.

In some embodiments, the non-cationic lipid in the lyophilized composition comprises from 20 mol % to 70 mol %, including but not limited to 20 mol %, 21 mol %, 22 mol %, 23 mol %, 24 mol %, 25 mol %, 26 mol %, 27 mol %, 28 mol %, 29 mol %, 30 mol %, 31 mol %, 32 mol %, 33 mol %, 34 mol %, 35 mol %, 36 mol %, 37 mol %, 38 mol %, 39 mol %, 40 mol %, 41 mol %, 42 mol %, 43 mol %, 44 mol %, 45 mol %, 46 mol %, 47 mol %, 48 mol %, 49 mol %, 50 mol %, 51 mol %, 52 mol %, 53 mol %, 54 mol %, 55 mol %, 56 mol %, 57 mol %, 58 mol %, 59 mol %, 60 mol %, 61 mol %, 62 mol %, 63 mol %, 64 mol %, 65 mol %, 66 mol %, 67 mol %, 68 mol %, 69 mol %, 70 mol %, or a value between any two of these numbers, of total lipids present in the lipid nanoparticles.

In some embodiments, the non-cationic lipid in the lyophilized composition comprises from 50 mol % to 65 mol % of total lipids present in the lipid nanoparticles.

In some embodiments, the non-cationic lipid in the lyophilized composition comprises from 40 mol % to 55 mol % of total lipids present in the lipid nanoparticles.

In some embodiments, the non-cationic lipid in the lyophilized composition comprises from 55 mol % to 62 mol % of total lipids present in the lipid nanoparticles.

In some embodiments, the non-cationic lipid in the lyophilized composition comprises from 30 mol % to 60 mol % of total lipids present in the lipid nanoparticles.

In some embodiments, the non-cationic lipid in the lyophilized composition comprises from 30 mol % to 50 mol % of total lipids present in the lipid nanoparticles.

In some embodiments, the non-cationic lipid in the lyophilized composition comprises from 20 mol % to 50 mol % of total lipids present in the lipid nanoparticles.

In some embodiments, the non-cationic lipid in the lyophilized composition comprises from 20 mol % to 40 mol % of total lipids present in the lipid nanoparticles.

In some embodiments, the non-cationic lipid in the present disclosure can be selected from the group consisting of a mixture of a phospholipid and cholesterol or a derivative thereof.

In some embodiments, the phospholipid in the present disclosure can be selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and a mixture thereof.

In some embodiments, the phospholipid in the present disclosure is 1,2-distearoyl-sn-glycero-3-phosphocholine (DSPC).

In some embodiments, the phospholipid in the present disclosure is 1,2-dioleoyl-sn-glycero-3-phosphoethanolamine (DOPE).

In some embodiments, the weight ratio of the phospholipid to the cationic lipid in the present disclosure ranges from 1:20 to 10:1; preferably, the weight ratio of the phospholipid to the cationic lipid ranges from 1:20 to 5:1; preferably, the weight ratio of the phospholipid to the cationic lipid ranges from 1:10 to 5:1; and more preferably, the weight ratio of the phospholipid to the cationic lipid ranges from 1:10 to 1:1.

In some embodiments, provided is the lyophilized composition, wherein a content of the phospholipid comprises from 5 mol % to 40 mol %, including but not limited to 5 mol %, 6 mol %, 7 mol %, 8 mol %, 9 mol %, 10 mol %, 11 mol %, 12 mol %, 13 mol %, 14 mol %, 15 mol %, 16 mol %, 17 mol %, 18 mol %, 19 mol %, 20 mol %, 21 mol %, 22 mol %, 23 mol %, 24 mol %, 25 mol %, 26 mol %, 27 mol %, 28 mol %, 29 mol %, 30 mol %, 31 mol %, 32 mol %, 33 mol %, 34 mol %, 35 mol %, 36 mol %, 37 mol %, 38 mol %, 39 mol %, 40 mol %, or a value between any two of these numbers, of total lipids present in the lipid nanoparticles.

In some embodiments, a content of the phospholipid in the lyophilized composition comprises from 5 mol % to 20 mol % of total lipids present in the lipid nanoparticles.

In some embodiments, a content of the phospholipid in the lyophilized composition comprises 15 mol % of total lipids present in the lipid nanoparticles.

In some embodiments, a content of the phospholipid in the lyophilized composition comprises 20 mol % of total lipids present in the lipid nanoparticles.

In some embodiments, a content of the phospholipid in the lyophilized composition comprises from 10 mol % to 20 mol % of total lipids present in the lipid nanoparticles.

In some embodiments, a content of the phospholipid in the lyophilized composition comprises 10 mol % of total lipids present in the lipid nanoparticles.

In some embodiments, the derivative of cholesterol includes, but are not limited to sitosterol, ergosterol, campesterol, stigmasterol, brassicasterol, tomatidine, and ursolic acid.

In another aspect, cholesterol or the derivative thereof in the lyophilized composition of the present disclosure comprises from 20 mol % to 60 mol %, including but not limited to 20 mol %, 21 mol %, 22 mol %, 23 mol %, 24 mol %, 25 mol %, 26 mol %, 27 mol %, 28 mol %, 29 mol %, 30 mol %, 31 mol %, 32 mol %, 33 mol %, 34 mol %, 35 mol %, 36 mol %, 37 mol %, 38 mol %, 39 mol %, 40 mol %, 41 mol %, 42 mol %, 43 mol %, 44 mol %, 45 mol %, 46 mol %, 47 mol %, 48 mol %, 49 mol %, 50 mol %, 51 mol %, 52 mol %, 53 mol %, 54 mol %, 55 mol %, 56 mol %, 57 mol %, 58 mol %, 59 mol %, 60 mol %, or a value between any two of these numbers, of total lipids present in the lipid nanoparticles.

In some embodiments, a content of cholesterol or the derivative thereof in the lyophilized composition comprises from 30 mol % to 60 mol % of total lipids present in the lipid nanoparticles.

In some embodiments, a content of cholesterol or the derivative thereof in the lyophilized composition comprises from 20 mol % to 45 mol % of total lipids present in the lipid nanoparticles.

In some embodiments, a content of cholesterol or the derivative thereof in the lyophilized composition comprises from 35 mol % to 45 mol % of total lipids present in the lipid nanoparticles.

In some embodiments, a content of cholesterol or the derivative thereof in the lyophilized composition comprises from 20 mol % to 35 mol % of total lipids present in the lipid nanoparticles.

In some embodiments, a content of cholesterol or the derivative thereof in the lyophilized composition comprises 40.5 mol % of total lipids present in the lipid nanoparticles.

In some embodiments, a content of cholesterol or the derivative thereof in the lyophilized composition comprises 45 mol % of total lipids present in the lipid nanoparticles.

In some embodiments, the molar ratio of the cholesterol or the derivative thereof to the phospholipid in the present disclosure ranges from 1.0 to 5.0, including but not limited to 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or a value between any two of these numbers.

In some embodiments, the molar ratio of the cholesterol or the derivative thereof to the phospholipid in the lipid nanoparticles of the present disclosure ranges from 2.0 to 5.0.

In another aspect, the lipid nanoparticles in the present disclosure further comprise at least one conjugated lipid, and the conjugated lipid includes, but is not limited to PEG-modified phosphatidylethanolamine, PEG-modified phosphatidic acid, PEG-modified ceramide, PEG-modified dialkylamine, PEG-modified diacylglycerol, and PEG-modified dialkylglycerol.

In some embodiments, the conjugated lipid is selected from the group consisting of distearoylphosphatidylethanolamine polyethylene glycol 2000 (DSPE-PEG2000), dimyristoylglycero-3-methoxypolyethylene glycol 2000 (DMG-PEG2000), and methoxypolyethylene glycol ditetradecylacetamide (ALC-0159).

In some embodiments, the conjugated lipid is selected from the group consisting of dimyristoylglycero-3-methoxypolyethylene glycol 2000.

In some embodiments, the conjugated lipid is selected from the group consisting of distearoylphosphatidylethanolamine polyethylene glycol 2000.

In some embodiments, the conjugated lipid inhibits the aggregation of lipid nanoparticles (particles).

In some embodiments, a content of the conjugated lipid in the lyophilized composition comprises from 0.5 mol % to 4 mol %, including but not limited to 0.5 mol %, 0.6 mol %, 0.7 mol %, 0.8 mol %, 0.9 mol %, 1.0 mol %, 1.2 mol %, 1.4 mol %, 1.6 mol %, 1.8 mol %, 2.0 mol %, 2.2 mol %, 2.4 mol %, 2.6 mol %, 2.8 mol %, 3.0 mol %, 3.2 mol %, 3.4 mol %, 3.6 mol %, 3.8 mol %, 4.0 mol %, or a value between any two of these numbers, of total lipids present in the lipid nanoparticles.

In some embodiments, a content of the conjugated lipid in the lyophilized composition comprises from 0.5 mol % to 1.0 mol % of total lipids present in the lipid nanoparticles.

In some embodiments, a content of the conjugated lipid in the lyophilized composition comprises from 1.5 mol % to 2.5 mol % of total lipids present in the lipid nanoparticles.

In some embodiments, a content of the conjugated lipid in the lyophilized composition comprises from 1 mol % to 2 mol % of total lipids present in the lipid nanoparticles.

In some embodiments, a content of the conjugated lipid in the lyophilized composition comprises 1.5 mol % of total lipids present in the lipid nanoparticles.

In some embodiments, a content of the conjugated lipid in the lyophilized composition comprises 2.0 mol % of total lipids present in the lipid nanoparticles.

In some embodiments, the weight ratio of the conjugated lipid to the cationic lipid ranges from 1:100 to 1:1; preferably, the weight ratio of the conjugated lipid to the cationic lipid ranges from 1:100 to 1:2; preferably, the weight ratio of the conjugated lipid to the cationic lipid ranges from 1:50 to 1:1; preferably, the weight ratio of the conjugated lipid to the cationic lipid ranges from 1:50 to 1:2; preferably, the weight ratio of the conjugated lipid to the cationic lipid ranges from 1:25 to 1:2; preferably, the weight ratio of the conjugated lipid to the cationic lipid ranges from 1:20 to 1:2.

In some embodiments, the lipid nanoparticles in the present disclosure comprise:
a cationic lipid comprising a compound of formula I or a pharmaceutically acceptable salt thereof, wherein the cationic lipid comprises from 10 mol % to 75 mol % of total lipids present in the lipid nanoparticles, 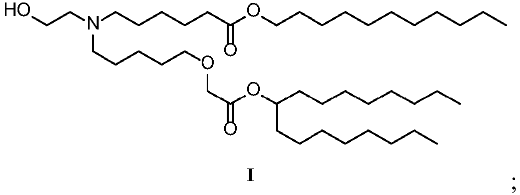
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lipid nanoparticles in the present disclosure comprise:
a cationic lipid comprising a compound of formula I or a pharmaceutically acceptable salt thereof, wherein the cationic lipid comprises from 20 mol % to 70 mol % of total lipids present in the lipid nanoparticles, 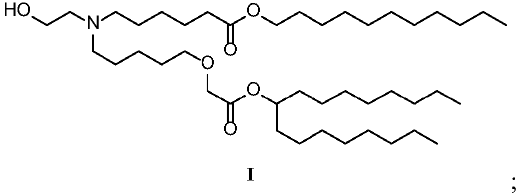
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lipid nanoparticles in the present disclosure comprise:
a cationic lipid comprising a compound of formula I or a pharmaceutically acceptable salt thereof, wherein the cationic lipid comprises from 10 mol % to 50 mol % of total lipids present in the lipid nanoparticles, 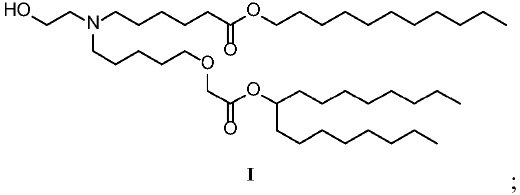
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 30 mol % to 60 mol % of total lipids present in the lipid nanoparticles; and
a conjugated lipid comprising from 0.5 mol % to 2 mol % of total lipids present in the lipid nanoparticles.

In another aspect, the lyophilized composition of the present disclosure further comprises a lyoprotectant.

In some embodiments, the lyoprotectant is selected from one or more of sucrose, trehalose, mannitol, and maltose; preferably, the lyoprotectant is selected from sucrose, or a combination of sucrose and trehalose.

In some embodiments, the lyoprotectant is sucrose.

In some embodiments, the lyoprotectant is a combination of trehalose and mannitol.

In some embodiments, the weight ratio of the lyoprotectant to the cationic lipid ranges from 200:1 to 1:1; preferably, the weight ratio of the lyoprotectant to the cationic lipid ranges from 150:1 to 1:1; preferably, the weight ratio of the lyoprotectant to the cationic lipid ranges from 100:1 to 1:1; preferably, the weight ratio of the lyoprotectant to the cationic lipid ranges from 100:1 to 20:1; preferably, the weight ratio of the lyoprotectant to the cationic lipid ranges from 800:1 to 20:1; preferably, the weight ratio of the lyoprotectant to the cationic lipid ranges from 50:1 to 20:1.

In some embodiments, the lyophilized composition of the present disclosure comprises the lyoprotectant at a concentration from 5% w/v to 20% w/v, and preferably at a concentration from 5% w/v to 15% w/v.

In some embodiments, the lyophilized composition of the present disclosure comprises sucrose at a concentration from 5% w/v to 20% w/v, preferably at a concentration from 5% w/v to 15% w/v, and preferably at a concentration from 8% w/v.

In some embodiments, the lyophilized composition of the present disclosure comprises trehalose at a concentration from 5% w/v to 20% w/v, and preferably at a concentration from 5% w/v to 15% w/v.

In some embodiments, the lyophilized composition of the present disclosure comprises sucrose at a concentration from 5% w/v to 10% w/v, and trehalose at a concentration from1% w/v to 10% w/v.

In another aspect, the lyophilized composition of the present disclosure further comprises a buffer.

In some embodiments, the buffer is selected from the group consisting of (N-morpholino)propanesulfonic acid (MOPS), 4-hydroxyethylpiperazine ethanesulfonic acid (HEPES), trihydroxymethylaminomethane (TRIS), 4-morpholinoethanesulfonic acid (MES), citrate, and phosphate buffered saline (PBS).

In some embodiments, the buffer is TRIS.

In some embodiments, the buffer has a concentration of about 10 mM to about 100 mM, preferably of about 15 mM to about 75 mM, and more preferably of about 10 mM to about 40 mM.

The present disclosure further provides a method for preparing the lyophilized composition, comprising the following steps:
a. providing a suspension of the lipid nanoparticles in a liquid medium;
b. adjusting the liquid medium to form a suspension containing NaCl; and
c. lyophilizing.

In some embodiments, in the preparation method of the present disclosure, the liquid medium further contains a lyoprotectant from 5% w/v to 20% w/v, and preferably from 5% w/v to 15% w/v.

In some embodiments, in the preparation method of the present disclosure, the liquid medium contains sucrose from 5% w/v to 20% w/v, and preferably from 5% w/v to 15% w/v.

In some embodiments, in the preparation method of the present disclosure, the liquid medium contains sucrose from 5% w/v to 10% w/v, and trehalose from 1% w/v to 10% w/v.

In some embodiments, in the preparation method of the present disclosure, the liquid medium contains 10-200 mM NaCl, preferably 10-150 mM NaCl, more preferably 20-150 mM NaCl, more preferably 20-120 mM NaCl, more preferably 50-150 mM NaCl, more preferably 50-120 mM NaCl, and more preferably 70-120 mM NaCl.

In another aspect, the lyophilized composition of the present disclosure does not comprise poloxamer.

In another aspect, the lyophilized composition of the present disclosure does not comprise potassium sorbate.

In some embodiments, the lipid nanoparticles in the present disclosure comprise:
a cationic lipid comprising from 10 mol % to 75 mol % of total lipids present in the lipid nanoparticles;
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lipid nanoparticles in the present disclosure comprise:
a cationic lipid comprising a compound of formula I or a pharmaceutically acceptable salt thereof, wherein the cationic lipid comprises from 10 mol % to 75 mol % of total lipids present in the lipid nanoparticles, 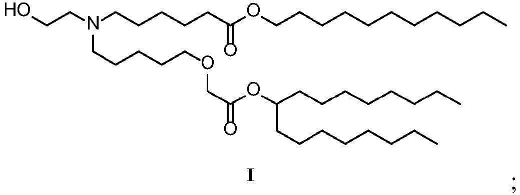
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lipid nanoparticles in the present disclosure comprise:
a cationic lipid comprising from 20 mol % to 70 mol % of total lipids present in the lipid nanoparticles;
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lipid nanoparticles in the present disclosure comprise:
a cationic lipid comprising a compound of formula I or a pharmaceutically acceptable salt thereof, wherein the cationic lipid comprises from 20 mol % to 70 mol % of total lipids present in the lipid nanoparticles, 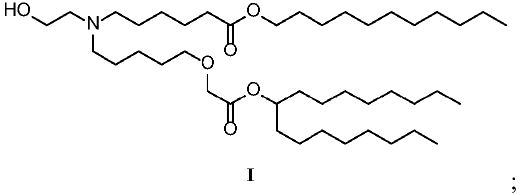
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lipid nanoparticles in the present disclosure comprise:
a cationic lipid comprising from 10 mol % to 50 mol % of total lipids present in the lipid nanoparticles;
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 30 mol % to 60 mol % of total lipids present in the lipid nanoparticles; and
a conjugated lipid comprising from 0.5 mol % to 2 mol % of total lipids present in the lipid nanoparticles.

In some embodiments, the lipid nanoparticles in the present disclosure comprise:
a cationic lipid comprising a compound of formula I or a pharmaceutically acceptable salt thereof, wherein the cationic lipid comprises from 10 mol % to 50 mol % of total lipids present in the lipid nanoparticles, 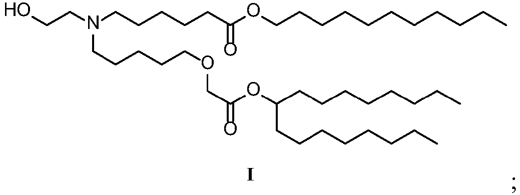
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 30 mol % to 60 mol % of total lipids present in the lipid nanoparticles; and
a conjugated lipid comprising from 0.5 mol % to 2 mol % of total lipids present in the lipid nanoparticles.

The lipid nanoparticles in the present disclosure further contain an active agent, and the active agent is selected from the group consisting of nucleic acids, including ribonucleic acids or deoxyribonucleic acids.

In some embodiments, the lyophilized composition of the present disclosure comprises:
an active agent including an mRNA;
sodium chloride, the weight ratio of sodium chloride to the cationic lipid ranging from 1:5 to 10:1;
a cationic lipid comprising from 10 mol % to 75 mol % of total lipids present in the lipid nanoparticles;
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lyophilized composition of the present disclosure comprises:
an active agent including an mRNA;
sodium chloride, the weight ratio of sodium chloride to the cationic lipid ranging from 1:5 to 10:1;
a cationic lipid comprising a compound of formula I or a pharmaceutically acceptable salt thereof, wherein the cationic lipid comprises from 10 mol % to 75 mol % of total lipids present in the lipid nanoparticles, 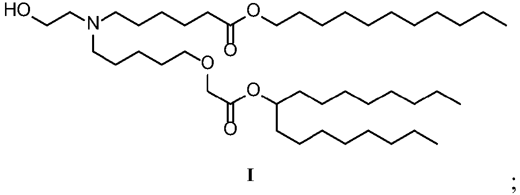
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lyophilized composition of the present disclosure comprises:
an active agent including an mRNA;
sodium chloride, the weight ratio of sodium chloride to the cationic lipid ranging from 1:5 to 10:1;
a cationic lipid comprising from 20 mol % to 70 mol % of total lipids present in the lipid nanoparticles;
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lyophilized composition of the present disclosure comprises:
an active agent including an mRNA;
sodium chloride, the weight ratio of sodium chloride to the cationic lipid ranging from 1:5 to 10:1;
a cationic lipid comprising a compound of formula I or a pharmaceutically acceptable salt thereof, wherein the cationic lipid comprises from 20 mol % to 70 mol % of total lipids present in the lipid nanoparticles, 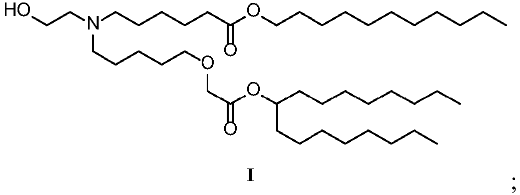
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
a conjugated lipid comprising from 0.5 mol % to 2 mol % of total lipids present in the lipid nanoparticles.

In some embodiments, the lyophilized composition of the present disclosure comprises:
an active agent including an mRNA;
sodium chloride, the weight ratio of sodium chloride to the cationic lipid ranging from 1:5 to 10:1;
a cationic lipid comprising from 10 mol % to 50 mol % of total lipids present in the lipid nanoparticles;
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 30 mol % to 60 mol % of total lipids present in the lipid nanoparticles; and
a conjugated lipid comprising from 0.5 mol % to 2 mol % of total lipids present in the lipid nanoparticles.

In some embodiments, the lyophilized composition of the present disclosure comprises:
an active agent including an mRNA;
sodium chloride, the weight ratio of sodium chloride to the cationic lipid ranging from 1:5 to 10:1;
a cationic lipid comprising a compound of formula I or a pharmaceutically acceptable salt thereof, wherein the cationic lipid comprises from 10 mol % to 50 mol % of total lipids present in the lipid nanoparticles, 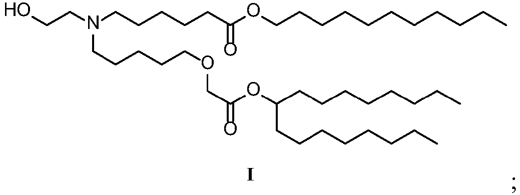
a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 30 mol % to 60 mol % of total lipids present in the lipid nanoparticles; and
a conjugated lipid comprising from 0.5 mol % to 2 mol % of total lipids present in the lipid nanoparticles.

In some embodiments, the lyophilized composition of the present disclosure comprises:
a) sodium chloride, the weight ratio of sodium chloride to the cationic lipid ranging from 1:5 to 10:1;
b) lipid nanoparticles comprising:
   an active agent including an mRNA;
   a cationic lipid comprising from 10 mol % to 75 mol % of total lipids present in the lipid nanoparticles;
   a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
   a conjugated lipid comprising from 0.5 mol % to 2 mol % of total lipids present in the lipid nanoparticles.

In some embodiments, the lyophilized composition of the present disclosure comprises:
a) sodium chloride, the weight ratio of sodium chloride to the cationic lipid ranging from 1:5 to 10:1;
b) lipid nanoparticles comprising:
   an active agent including an mRNA;
   a cationic lipid comprising a compound of formula I or a pharmaceutically acceptable salt thereof, wherein the cationic lipid comprises from 10 mol % to 75 mol % of total lipids present in the lipid nanoparticles, 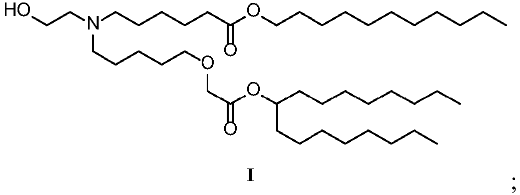
   a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
   a conjugated lipid comprising from 0.5 mol % to 2 mol % of total lipids present in the lipid nanoparticles.

In some embodiments, the lyophilized composition of the present disclosure comprises:
a) sodium chloride, the weight ratio of sodium chloride to the cationic lipid ranging from 1:5 to 10:1;
b) lipid nanoparticles comprising:
   an active agent including an mRNA;
   a cationic lipid comprising from 20 mol % to 70 mol % of total lipids present in the lipid nanoparticles;
   a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
   a conjugated lipid comprising from 0.5 mol % to 2 mol % of total lipids present in the lipid nanoparticles.

In some embodiments, the lyophilized composition of the present disclosure comprises:
a) sodium chloride, the weight ratio of sodium chloride to the cationic lipid ranging from 1:5 to 10:1;
b) lipid nanoparticles comprising:
   an active agent including an mRNA;
   a cationic lipid comprising a compound of formula I or a pharmaceutically acceptable salt thereof, wherein the cationic lipid comprises from 20 mol % to 70 mol % of total lipids present in the lipid nanoparticles, 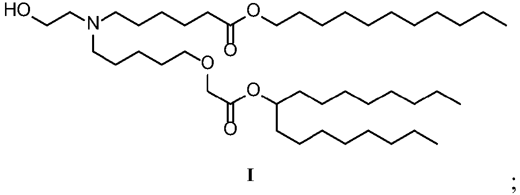
   a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 15 mol % to 60 mol % of total lipids present in the lipid nanoparticles; and
   a conjugated lipid comprising from 0.5 mol % to 2 mol % of total lipids present in the lipid nanoparticles.

In some embodiments, the lyophilized composition of the present disclosure comprises:
a) sodium chloride, the weight ratio of sodium chloride to the cationic lipid ranging from 1:5 to 10:1;
b) lipid nanoparticles comprising:
   an active agent including an mRNA;
   a cationic lipid comprising from 10 mol % to 50 mol % of total lipids present in the lipid nanoparticles;
   a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 30 mol % to 60 mol % of total lipids present in the lipid nanoparticles; and
   a conjugated lipid comprising from 0.5 mol % to 2 mol % of total lipids present in the lipid nanoparticles.

In some embodiments, the lyophilized composition of the present disclosure comprises:
a) sodium chloride, the weight ratio of sodium chloride to the cationic lipid ranging from 1:5 to 10:1;
b) lipid nanoparticles comprising:
   an active agent including an mRNA;
   a cationic lipid comprising a compound of formula I or a pharmaceutically acceptable salt thereof, wherein the cationic lipid comprises from 10 mol % to 50 mol % of total lipids present in the lipid nanoparticles, 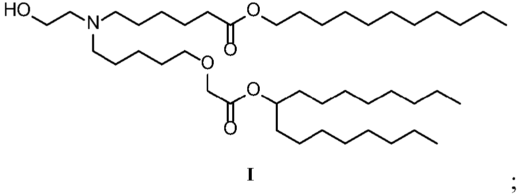
   a non-cationic lipid selected from the group consisting of a phospholipid and cholesterol or a derivative thereof, wherein the phospholipid comprises from 5 mol % to 40 mol % of total lipids present in the lipid nanoparticles, and the cholesterol or the derivative thereof comprises from 30 mol % to 60 mol % of total lipids present in the lipid nanoparticles; and
   a conjugated lipid comprising from 0.5 mol % to 2 mol % of total lipids present in the lipid nanoparticles.

In some embodiments, the lyophilized composition comprises a lyoprotectant at a concentration from 5% w/v to 20% w/v, and preferably 5% w/v to 15% w/v.

In some embodiments, the lyophilized composition comprises a lyoprotectant, wherein the lyoprotectant is sucrose of 5% w/v to 20% w/v, preferably of 5% w/v to 15% w/v, and more preferably of 5% w/v to 10% w/v.

In some embodiments, the lyophilized composition comprises a lyoprotectant, wherein the lyoprotectant comprises sucrose of 5% w/v to 10% w/v and trehalose of 1% w/v to 10% w/v, and preferably sucrose of 5% w/v to 10% w/v and trehalose of 2% w/v to 8% w/v.

In some embodiments, the lyophilized composition has the active agent at an amount of about 0.01 mg/mL to about 1mg/mL, about 0.05 mg/mL to about 0.5mg/mL, about 0.1 mg/mL to about 0.5 mg/mL, or about 0.2 mg/mL, about 0.05 mg/mL, or about 0.02 mg/mL.

In some embodiments, the weight ratio of the active agent to the cationic lipid ranges from 1:10 to 1:60; preferably, the weight ratio of the active agent to the cationic lipid ranges from 1:10 to 1:50; and preferably, the weight ratio of the active agent to the cationic lipid ranges from 1:10 to 1:40.

In some embodiments, the active agent is selected from the group consisting of any one or any combination of a small interfering RNA (siRNA), a microRNA (miRNA), a small hairpin RNA (shRNA), and a messenger RNA (mRNA).

In some embodiments, the active agent is an mRNA.

In some embodiments, the mRNA comprises an ORF, and the ORF expresses a protein or polypeptide.

In some embodiments, the mRNA encodes a protein or polypeptide.

In some embodiments, the mRNA encodes an antigen.

In some embodiments, the protein or polypeptide encoded by the mRNA is derived from an infectious agent, or is an infectious agent antigen.

In some embodiments, the protein or polypeptide is a viral antigen and/or a bacterial antigen.

In some embodiments, the virus is selected from the group consisting of adenoviruses, herpesviruses (e.g., herpes simplex virus type I, herpes simplex virus type II, and human herpesvirus type 8), encephalitis viruses, papillomaviruses, varicella zoster virus (VZV), Epstein-Barr virus, human cytomegalovirus, human papillomavirus, BK virus, JC virus, smallpox, poliovirus, hepatitis viruses (e.g., hepatitis A, B, C, D, or E virus), human bocaviruses, parvoviruses (e.g., B19), human astroviruses, Norwalk virus, coxsackieviruses, rhinoviruses, severe acute respiratory syndrome virus, yellow fever virus, dengue virus, West Nile virus, rubella virus, human immunodeficiency viruses (HIV), influenza A or B virus, Guanarito virus, Junin virus, Lassa virus, Machupo virus, Sabia virus, Crimean-Congo hemorrhagic fever virus, ebolaviruses, Marburg virus, measles virus, mumps virus, parainfluenza viruses, respiratory syncytial virus (RSV), human metapneumovirus, Hendra virus, Nipah virus, rabies virus, rotaviruses, circoviruses, Colorado tick fever virus, Hantaan virus, Middle East respiratory coronavirus, Chikungunya virus, or Banna virus.

In some embodiments, the virus is selected from the group consisting of an influenza virus (e.g., influenza A virus, influenza B virus), a coronavirus, and a combination thereof. For example, the coronavirus is SARS-COV-2; the coronavirus antigen is a spike protein; the spike protein is selected from the group consisting of a spike protein of any one of the viral strain SARS-COV-2, SARS-COV-2 Alpha, SARS-COV-2 Beta, SARS-COV-2 Gamma, SARS-COV-2 Kappa, SARS-COV-2 Delta or SARS-COV-2 Omicron. For example, the influenza virus is selected from the group consisting of influenza A virus and influenza B virus; the influenza virus antigen is a hemagglutinin protein (HA) and/or a neuraminidase (NA); the influenza virus antigen is selected from a hemagglutinin protein and/or a neuraminidase of any one of the viral strains influenza A virus H1N1, influenza A virus H3N2, influenza B virus Victoria, and influenza B virus Yamagata.

In some embodiments, the virus is RSV. For example, the RSV antigen is a RSV attachment protein (G) or an immunogenic fragment thereof, a RSV fusion (F) glycoprotein or an immunogenic fragment thereof, or a combination thereof; for example, the RSV antigen is an unfused glycoprotein F. The RSV antigenic polypeptides or immunogenic fragments thereof and sequences thereof disclosed in WO2017070622A and the unfused glycoproteins F and sequences thereof disclosed in WO2014160463A, WO2012158613A, WO2017109629A, and WO2017172890A are incorporated herein by reference in their entirety.

In some embodiments, the virus is VZV. For example, the VZV antigen is a VZV glycoprotein. For example, the VZV glycoprotein may be a VZV gE, gI, gB, gH, gK, gL, gC, gN, or gM polypeptide or an immunogenic fragment or epitope thereof. The VZV antigenic polypeptides and sequences thereof disclosed in WO2017070601A are incorporated herein by reference in their entirety.

In some embodiments, the protein or polypeptide is selected from the group consisting of fructose-bisphosphate aldolase A (ALDOA), α-methylacyl-CoA racemase (AMACR), serum amyloid P component (APCS), angiopoietin 1 (ANGPT1), apolipoprotein A-I (APOA1) Milano, apolipoprotein A-I (APOA1) Paris, apolipoprotein A-I (APOA1), argininosuccinate lyase (ASL), artemin (ARTN), arylsulfatase B (ARSB), bactericidal/permeability-increasing protein (rBPI-21), bone morphogenetic protein 2 (BMP2), bone morphogenetic protein 7 (BMP7), branched-chain keto acid dehydrogenase E1 α polypeptide (BCKDHA), colony stimulating factor 2 (granulocyte-macrophage) (GM-CSF), colony stimulating factor 3 (granulocyte) (GCSF), deoxyribonuclease I (DNase 1), erythropoietin (EPO), factor IX, factor VII, factor XI, fibrinogen A (FGA), fibroblast growth factor 18 (FGF18), fibroblast growth factor 23 (FGF23), fibroblast growth factor 7 (FGF7 or KGF), follistatin (FST), fumarylacetoacetate hydrolase (fumarylacetoacetase) (FAH), galactokinase 1 (GALK1), galactosidase α (GLA), glucan (1,4-α-) branching enzyme 1 (GBE1), glycoprotein hormone α polypeptide (CGA or FSH-α), hemoglobin β (HBB), hepatocyte growth factor (HGF), human growth hormone (hGH), insulin aspart, insulin glargine, insulin glulisine, insulin lispro, interferon β (IFNB), interferon α2 (IFNA2), interleukin 10 (IL-10), interleukin 15 (IL-15), interleukin 7 (IL-7), klotho (KL), lecithin-cholesterol acyltransferase (LCAT), lysosomal acid lipase A cholesterol esterase (LIPA), lipoprotein lipase (LPL), low-density lipoprotein receptor (LDLR), mannosidase α class 2B member 1 (MAN2B1), microsomal triglyceride transfer protein (MTTP), N-acetylglutamate synthase (NAGS), neuregulin 1 (NRG1), ornithine transcarbamylase (OTC), phosphorylase kinase α 2 (liver) (PHKA2), plasminogen (PLAT), septin-4 (ARTS or SEPT4), serpin peptidase inhibitor clade C (antithrombin) member 1 (SERPINC1), serpin peptidase inhibitor clade F (α-2 antiplasmin pigment epithelium derived factor) member 2 (SERPINF2), sirtuin 1 (SIRT1), sirtuin 6 (SIRT6), solute lipid nanoparticle family 16 member 3 (monocarboxylate transporter 4) (SLC16A3), solute lipid nanoparticle family 2 (facilitated glucose transporter) member 1 (SLC2A1 or GLUT1), sortilin 1 (SORT1), thrombopoietin (THPO), transforming growth factor β (TGFB1), tuftelin 1 (TUFT1), tumor protein p53 (TP53), tyrosinase (TYR), UDP glucuronosyltransferase 1 family polypeptide A1 (UGT1A1), vascular endothelial growth factor (VEGF), X-linked inhibitor of apoptosis (XIAP), and any combination thereof. The mRNA-encoded target polypeptides and sequences thereof disclosed in WO2013151736A are incorporated herein by reference in their entirety. In some embodiments, the protein or polypeptide is hepatocyte growth factor (HGF). In some embodiments, the HGF is human hepatocyte growth factor (hHGF). In some specific embodiments, the amino acid sequence of the HGF has the GeneBank accession number NP_000592.3, and is set forth in SEQ ID NO: 1. In some specific embodiments, an ORF sequence expressing the HGF is a nucleic acid sequence or codon-optimized nucleic acid sequence encoding the amino acid sequence set forth in SEQ ID NO: 1.

Amino acid sequence of HGF:

MWVTKLLPALLLQHVLLHLLLLPIAIPYAEGQRKRRNTIHEFKKSAKTTLI
KIDPALKIKTKKVNTADQCANRCTRNKGLPFTCKAFVFDKARKQCLWFPFNS
MSSGVKKEFGHEFDLYENKDYIRNCIIGKGRSYKGTVSITKSGIKCQPWSSMIP
HEHSFLPSSYRGKDLQENYCRNPRGEEGGPWCFTSNPEVRYEVCDIPQCSEVE
CMTCNGESYRGLMDHTESGKICQRWDHQTPHRHKFLPERYPDKGFDDNYCR
NPDGQPRPWCYTLDPHTRWEYCAIKTCADNTMNDTDVPLETTECIQGQGEG
YRGTVNTIWNGIPCQRWDSQYPHEHDMTPENFKCKDLRENYCRNPDGSESP
WCFTTDPNIRVGYCSQIPNCDMSHGQDCYRGNGKNYMGNLSQTRSGLTCSM
WDKNMEDLHRHIFWEPDASKLNENYCRNPDDDAHGPWCYTGNPLIPWDYC
PISRCEGDTTPTIVNLDHPVISCAKTKQLRVVNGIPTRTNIGWMVSLRYRNKHI
CGGSLIKESWVLTARQCFPSRDLKDYEAWLGIHDVHGRGDEKCKQVLNVSQL
VYGPEGSDLVLMKLARPAVLDDFVSTIDLPNYGCTIPEKTSCSVYGWGYTGLI
NYDGLLRVAHLYIMGNEKCSQHHRGKVTLNESEICAGAEKIGSGPCEGDYGG
PLVCEQHKMRMVLGVIVPGRGCAIPNRPGIFVRVAYYAKWIHKIILTYKVPQS
SEQ ID NO:1

In some embodiments, the protein or polypeptide is a cancer antigen. The cancer antigen includes, but is not limited to one or more traditional cancer antigens or subject-specific cancer antigens (including RNAs encoding one or more known cancer antigens specific for the tumor or cancer antigens specific for each subject; the cancer antigens are referred to as neo-epitopes or subject-specific epitopes or antigens). In some specific embodiments, the cancer antigen is a subject-specific cancer antigen. In some specific embodiments, the subject-specific cancer antigen may represent an exome of a tumor sample of the subject, or a transcriptome of a tumor sample of the subject. In some embodiments, the subject-specific cancer antigen may represent an exosome of the subject. The subject-specific cancer antigens and identification methods therefore described in WO2012159754A and WO2017070618A are incorporated herein by reference in their entirety. In some specific embodiments, the cancer antigen is a traditional cancer antigen. In some embodiments, the traditional cancer antigen is a non-mutated antigen. In some specific embodiments, the traditional cancer antigen is a mutated antigen. For example, cancer antigens may be carcinoembryonic antigen, α1-fetoprotein, isoferritin, fetal sulphoglycoprotein, α2-H-ferritin, and γ-fetoprotein.

In some embodiments, the protein or polypeptide is an antibody or an antigen-binding fragment thereof. For example, the protein or polypeptide is an anti-PD-1 antibody or an antigen-binding fragment thereof. In some specific embodiments, the antibody or the antigen-binding fragment thereof is, for example, a camelid antibody, a chimeric antibody, a humanized antibody, a fully human antibody, or an antigen-binding fragment thereof. In some specific embodiments, the antibody or the antigen-binding fragment thereof is, for example, a recombinant antibody or a fragment thereof. In some specific embodiments, the antibody or the antigen-binding fragment thereof is a linear antibody, a single-chain antibody, a nanobody, a peptibody, a domain antibody, or a multispecific antibody (a bispecific antibody, a diabody, a triabody, a tetrabody, a tandem di-scFv, a tandem tri-scFv). In some specific embodiments, the anti-PD-1 antibody or the antigen-binding fragment thereof has a heavy chain amino acid sequence set forth in SEQ ID NO: 2, and a light chain amino acid sequence set forth in SEQ ID NO: 3. In some specific embodiments, an ORF sequence expressing the anti-PD-1 antibody or the antigen-binding fragment thereof comprises any one selected from the group consisting of the following 1)-2):
1) a nucleic acid sequence or codon-optimized nucleic acid sequence encoding the heavy chain amino acid sequence set forth in SEQ ID NO: 2, and a nucleic acid sequence or codon-optimized nucleic acid sequence encoding the light chain amino acid sequence set forth in SEQ ID NO: 3;
2) nucleic acid sequences or codon-optimized nucleic acid sequences encoding a HCDR1, a HCDR2 and a HCDR3 in the heavy chain amino acid sequence set forth in SEQ ID NO: 2, and nucleic acid sequences or codon-optimized nucleic acid sequences encoding a LCDR1, a LCDR2 and a LCDR3 in the light chain amino acid sequence set forth in SEQ ID NO: 3, the CDRs being defined according to the Kabat, IMGT, Chothia, AbM, or Contact numbering scheme, for example, according to the Kabat numbering scheme.
   Heavy chain amino acid sequence of PD-1 antibody 
MEWSWVFLFFLSVTTGVHSEVQLVESGGGLVQPGGSLRLSCAASGFTFSS
YMMSWVRQAPGKGLEWVATISGGGANTYYPDSVKGRFTISRDNAKNSLYLQ
 
MNSLRAEDTAVYYCARQLYYFDYWGQGTTVTVSSASTKGPSVFPLAPCSRST
SESTAALGCLVKDYFPEPVTVSWNSGALTSGVHTFPAVLQSSGLYSLSSVVTVP
SSSLGTKTYTCNVDHKPSNTKVDKRVESKYGPPCPPCPAPEFLGGPSVFLFPPK
PKDTLMISRTPEVTCVVVDVSQEDPEVQFNWYVDGVEVHNAKTKPREEQFNS
TYRVVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLDSDGS
FFLYSRLTVDKSRWQEGNVFSCSVMHEALHNHYTQKSLSLSLGK
SEQ ID NO:2
   Light chain amino acid sequence of PD-1 antibody

MSVPTQVLGLLLLWLTDARCDIQMTQSPSSLSASVGDRVTITCLASQTIGT
WLTWYQQKPGKAPKLLIYTATSLADGVPSRFSGSGSGTDFTLTISSLQPEDFAT
YYCQQVYSIPWTFGGGTKVEIKRTVAAPSVFIFPPSDEQLKSGTASVVCLLNNF
YPREAKVQWKVDNALQSGNSQESVTEQDSKDSTYSLSSTLTLSKADYEKHK
VYACEVTHQGLSSPVTKSFNRGEC
SEQ ID NO:3

In some embodiments, the mRNA comprises:
(a) an open reading frame (ORF),
(b) an untranslated region element (UTR), and
(c) a polyadenylic acid (poly-A) tail,
   wherein (b) the untranslated region element (UTR) may include (b1) a 5'UTR and/or (b2) a 3'UTR;
   wherein (b) the untranslated region element (UTR) and (c) the polyadenylic acid (poly-A) tail may be present simultaneously or absent simultaneously, or one of them is present.

In some embodiments, the 5'UTR is selected from the group consisting of a β-globin 5'UTR, a 5'UTR containing a strong Kozak translational initiation signal, a cytochrome b-245α polypeptide (CYBA) 5'UTR, a hydroxysteroid (17-β) dehydrogenase (HSD17B4) 5'UTR, a tobacco etch virus (TEV) 5'UTR, a heat shock protein 70 (Hsp70) 5'UTR, or an eIF4G 5'UTR.

In some embodiments, the 3'UTR is selected from the group consisting of an α-globin 3'UTR, a β-actin 3'UTR, a CYBA 3'UTR, an albumin 3'UTR, a growth hormone (GH) 3'UTR, a VEEV 3'UTR, or a hepatitis B virus (HBV) 3'UTR.

In some embodiments, the poly-A tail is selected from the group consisting of A120, A30L70, HGH polyA, SV40polyA, BGH polyA, rbGlob polyA, or SV40late polyA.

In some embodiments, the mRNA comprises modified nucleotides or nucleosides. The modified nucleotides and nucleosides may be naturally occurring modified nucleotides and nucleosides or non-naturally occurring modified nucleotides and nucleosides. The modifications can include those at the sugar, backbone, or nucleobase portion of the nucleotide and/or nucleoside that are recognized in the art. In some specific embodiments, modified nucleobases in the mRNA comprise 1-methyl-pseudouridine (m1ψ), 1-ethyl-pseudouridine (e1ψ), 5-methoxy-uridine (mo5U), 5-methyl-cytidine (m5C), and/or pseudouridine (ψ). In some specific embodiments, modified nucleobases in the mRNA comprise 5-methoxymethyluridine, 5-methylthiouridine, 1-methoxymethylpseudouridine, 5-methylcytidine, and/or 5-methoxycytidine. In some specific embodiments, the mRNA comprises a combination of at least two (e.g., 2, 3, 4, or more) of any of the aforementioned modified nucleobases, including but not limited to chemical modifications.

In some embodiments, the mRNA further comprises a 5'Cap structure. In some specific embodiments, the 5'Cap structure is located upstream of the 5'UTR, e.g., at the 5' end of the 5'UTR. In some specific embodiments, the 5'Cap structure is a cap structure known to those skilled in the art, such as Cap0 (methylation to the first nucleobase, e.g., m7GpppN), Cap1 (additional methylation to the ribose of a nucleotide adjacent to m7GpppN, e.g., m7G(5')ppp(5')(2'OMeA)pG), Cap2 (additional methylation to the ribose of the 3rd nucleotide downstream of m7GpppN), Cap3 (additional methylation to the ribose of the 3rd nucleotide downstream of m7GpppN), Cap4 (additional methylation to the ribose of the 4th nucleotide downstream of m7GpppN), ARCA (anti-reverse cap analog), modified ARCA (e.g., phosphorothioate-modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In another aspect, the lipid nanoparticles in the present disclosure have an average particle size ranging from 10 nm to 300 nm.

In some embodiments, the lipid nanoparticles have an average particle size ranging from 10 nm to 250 nm.

In some embodiments, the lipid nanoparticles have an average particle size ranging from 20 nm to 200 nm.

In some embodiments, the lipid nanoparticles have an average particle size ranging from 50 nm to 200 nm.

In some embodiments, the lipid nanoparticles have an average particle size ranging from 50 nm to 150 nm.

In some embodiments, the lipid nanoparticles have an average particle size ranging from 50 nm to 140 nm.

In some embodiments, the lipid nanoparticles have an average particle size ranging from 50 nm to 130 nm.

In some embodiments, after the reconstitution of the lyophilized composition of the present disclosure, the average particle size of the lipid nanoparticles increases by no more than 100%. In some embodiments, the average particle size of the lipid nanoparticles increases by no more than 80%. In some embodiments, the average particle size of the lipid nanoparticles increases by no more than 60%.

In some embodiments, the lyophilized composition of the present disclosure is tested for mRNA integrity immediately after being removed from the lyophilization chamber, wherein the mRNA integrity is not less than 75%, or not less than 80%, or not less than 85%, or not less than 86%, or not less than 87%, or not less than 88%, or not less than 89%, or not less than 90%. The immediate testing is preformed within 1 hour after removal from the chamber. In some embodiments, the lyophilized composition of the present disclosure is tested for mRNA integrity after being placed at 37°C for 72 h after removal from the chamber, wherein the mRNA integrity is not less than 75%, or not less than 80%, or not less than 85%, or not less than 86%, or not less than 87%, or not less than 88%, or not less than 89%, or not less than 90%.

The present disclosure further provides a reconstituted solution obtained by a step of reconstituting the aforementioned lyophilized composition or a lyophilized composition prepared by the aforementioned preparation method, wherein a solvent for the reconstitution is water for injection.

The present disclosure further provides use of the aforementioned lyophilized composition or reconstituted solution in the preparation of a medicament for inducing a protective immune response in a vertebrate.

The present disclosure further provides use of the lyophilized composition or the reconstituted solution in the preparation of a medicament for treating a disease or dysfunction in a mammal. In some embodiments, the disease is preferably selected from the group consisting of viral infections (e.g., a disease caused by coronavirus, influenza virus, HIV, or respiratory syncytial virus), liver disease, cancer, herpes, ischemic disease, metabolic syndrome, diabetes and its complication, restenosis, or nerve injury. In some embodiments, the ischemic disease is selected from the group consisting of coronary artery disease (CAD), peripheral arterial disease (PAD), myocardial infarction, limb ischemia, thromboangiitis obliterans (TAO), and diabetic arteriosclerosis obliterans (DAO). In some embodiments, the limb ischemia is lower limb ischemia, for example severe lower limb ischemia (CLI). In some embodiments, the diabetes and its complication are selected from the group consisting of diabetic peripheral neuropathy, diabetic foot ulcer (DFU), and diabetic arteriosclerosis obliterans (DAO).

### Terminology

The term "lipid" refers to a class of organic compounds that include, but are not limited to esters of fatty acids, and are characterized by being insoluble in water but soluble in many organic solvents. They are usually divided into at least three categories: (1) "simple lipids", including fats, oils and waxes; (2) "compound lipids", including phospholipids and glycolipids; and (3) "derived lipids", such as steroids.

The term "cationic lipid" refers to any one of a number of lipid species that carry a net positive charge at a selected pH, such as a physiological pH (e.g., pH of about 7.0).

The term "anionic lipid" refers to any lipid that is negatively charged at a physiological pH. These lipids include, but are not limited to phosphatidylglycerol, cardiolipin, diacylphosphatidylserine, diacylphosphatidic acid, N-dodecanoylphosphatidylethanolamine, N-succinylphosphatidylethanolamine, N-glutarylphosphatidylethanolamine, lysylphosphatidylglycerol, palmitoyloleoylphosphatidylglycerol (POPG), and other anionic modifying groups linked to neutral lipids.

The term "neutral lipid" refers to any one of a number of lipid species that exist in an uncharged or neutral zwitterionic form at a selected pH. At a physiological pH, such lipids include, for example, diacylphosphatidylcholine, diacylphosphatidylethanolamine, ceramide, sphingomyelin, cephalin, cholesterol, cerebroside, and diacylglycerol.

The term "amphiphilic lipid" refers, in part, to any suitable material in which the hydrophobic portion of the lipid material is oriented into a hydrophobic phase and the hydrophilic portion is oriented into an aqueous phase. The hydrophilicity results from the presence of polar or charged groups such as sugars, phosphate radical, carboxyl, sulfate radical, amino, sulfhydryl, nitro, hydroxy, and other similar groups. Hydrophobicity can be conferred by the inclusion of nonpolar groups that include, but are not limited to long-chain saturated and unsaturated aliphatic hydrocarbon groups, and groups substituted with one or more aromatic, cycloaliphatic or heterocyclic groups. Examples of amphiphilic compounds include, but are not limited to phospholipids, aminolipids, and sphingolipids.

The term "non-cationic lipid" refers to any amphiphilic lipid and any other neutral or anionic lipid.

The term "conjugated lipid" refers to a conjugated lipid that inhibits the aggregation of lipid particles. The conjugated lipid includes, but is not limited to PEG-modified phosphatidylethanolamine, PEG-modified phosphatidic acid, PEG-modified ceramide, PEG-modified dialkylamine, PEG-modified diacylglycerol, and PEG-modified dialkylglycerol.

The term "mammal" refers to any mammalian species, such as a human, mouse, rat, dog, cat, hamster, guinea pig, rabbit, and livestock.

The term "nucleic acid" as used herein refers to a polymer containing at least two deoxyribonucleotides or ribonucleotides in a single-stranded or double-stranded form, and includes DNA and RNA. DNA may be in the form of, for example, antisense molecules, plasmid DNA, pre-concentrated DNA, PCR products, lipid nanoparticles (P1, PAC, BAC, YAC, artificial chromosomes), expression cassettes, chimeric sequences, chromosomal DNA, or derivatives and combinations of these groups. RNA may be in the form of small interfering RNA (siRNA), microRNA (miRNA), small hairpin RNA (shRNA), and messenger RNA (mRNA).

The term "antigen" encompasses any substance that will elicit an immune response. In particular, "antigen" relates to any substance, preferably a peptide or protein that specifically reacts with antibodies or T lymphocytes (T cells). In the present disclosure, the term "antigen" includes any molecule that comprises at least one epitope. Preferably, an antigen herein is a molecule that (optionally after processing) induces an immune response that is preferably specific for the antigen (including cells that express the antigen). Herein, any suitable antigen that is a candidate for an immune response may be used, wherein the immune response is preferably a cellular immune response. In the context of some embodiments, the antigen is preferably presented by cells, preferably by antigen-presenting cells (including diseased cells, particularly cancer cells, in the context of MHC molecules), which results in an immune response against the antigen. Preferably, the antigen is a product corresponding to or derived from a natural antigen. Such natural antigens include tumor antigens.

The term "cancer antigen" is used interchangeably with "tumor antigen" or "tumor-associated antigen", and it is capable of stimulating an immune response, preferably a cellular response against the antigen or cells characterized by expressing the antigen and preferably presenting the antigen (e.g., diseased cells, particularly cancer cells). In some embodiments, "cancer antigen" is specifically expressed under normal conditions in a limited number of tissues and/or organs or at specific developmental stages (for example, the tumor antigen may be specifically expressed under normal conditions in gastric tissues (preferably in the gastric mucosa), in reproductive organs (e.g., in a testis), in trophoblastic tissues (e.g., in the placenta), or in germline cells), and is expressed or abnormally expressed in one or more tumors or cancer tissues. In some embodiments, "a limited number" means being not more than 3, e.g., not more than 2.

The term "vaccine" relates to a pharmaceutical preparation (lyophilized composition) or product that, upon administration, induces an immune response, in particular a cellular immune response, that recognizes and attacks pathogens (including viruses or bacteria) or diseased cells such as cancer cells. A vaccine can be used to prevent or treat a disease, for example, to prevent viral infection or to treat cancer.

The term "5' untranslated region element" (5'UTR) refers to a non-coding polypeptide mRNA region located directly upstream (i.e., 5') of the start codon (i.e., the first codon of an mRNA transcript translated by a ribosome).

The term "3' untranslated region element" (3'UTR) refers to a non-coding polypeptide mRNA region located directly downstream (i.e., 3') of the stop codon (i.e., the codon indicating the termination of translation in the mRNA transcript).

The term "open reading frame" (ORF) refers to a continuous stretch of DNA that begins with a start codon (e.g., methionine (ATG)), ends with a stop codon (e.g., TAA, TAG, or TGA), and encodes a protein or polypeptide.

The term "polyadenylic acid (polyA) tail" refers to an mRNA region located downstream, e.g., directly downstream (i.e., 3'), of a 3'UTR that contains multiple consecutive adenosine monophosphates. A polyadenylic acid tail may contain 10 to 300 adenosine monophosphates. For example, a polyadenylic acid tail may contain 10, 20, 30, 40, 50, 60, 70, 80, 90, 100, 110, 120, 130, 140, 150, 160, 170, 180, 190, 200, 210, 220, 230, 240, 250, 260, 270, 280, 290, or 300 adenosine monophosphates. In some embodiments, a polyadenylic acid tail contains 50 to 250 adenosine monophosphates. In a relevant biological setting (e.g., in cells, *in vivo*), the poly(adenylic acid) tail functions to protect mRNA from enzymatic degradation, e.g., in the cytoplasm, and aids in transcription termination and/or nuclear export of the mRNA for translation.

The "average particle size" (Z-average size) described in the present disclosure, e.g., "an average particle size of less than 2000 nm", is an average light intensity value calculated from the light intensities contributed by different types of particles. The "average particle size" can be an average particle size of particles measured using conventional particle size measurement techniques well known to those skilled in the art. Such techniques include, for example, sedimentation field flow fractionation, photon correlation spectroscopy, light scattering, etc.

The polydispersity index "PDI" of the present disclosure reflects the degree of uniformity of the particle size of particles, and is an important index for particle size characterization.

The terms "mixed into" and "mixed" mean that the order of addition of the components is not limited. For example, when A is mixed into B, it can mean either A is added to B or B is added to A; when A and B are mixed, it can mean either A is added to B and mixed or B is added to A and mixed.

The numerical values in the present disclosure are instrument measurements and are subject to a certain degree of error. Generally, ±10% is within a reasonable error range. However, the context in which these values are used must be considered. For example, regarding the particle size of the active ingredient, the value represents a measurement error variation not exceeding ±10%, and may be ±9%, ±8%, ±7%, ±6%, ±5%, ±4%, ±3%, ±2%, or ±1%, preferably ±5%.

The compound of formula I of the present disclosure is prepared with reference to the method in WO2023125738A, and the relevant contents are incorporated herein by reference for illustrative purposes.

The mRNA used for testing in the present disclosure is luciferase mRNA.

The present disclosure provides lyophilized lipid nanoparticles with a stable particle size before and after lyophilization and reconstitution, with an average particle size not exceeding 150 nm, thereby avoiding immune responses caused by lipid nanoparticles.

### DESCRIPTION OF THE DRAWINGS

Figure 1 shows the particle sizes of Formulations 1-4 in different buffer solutions before lyophilization.
Figure 2 shows the particle sizes of Formulations 1-4 after lyophilization and reconstitution.
Figure 3 shows the rates of particle size change of Formulations 1-4 before and after lyophilization.
Figure 4 shows the mRNA integrity of Formulations 1-4 after removal from the lyophilization chamber.
Figure 5 shows the mRNA integrity of Formulations 7-9 after removal from the lyophilization chamber.
Figure 6 shows the mRNA integrity of Formulations 1-4 under 37°C high-temperature storage conditions.
Figure 7 shows the mRNA integrity of Formulations 7-9 under 37°C high-temperature storage conditions.
Figure 8 shows the fluorescence intensity values of expression levels in the cells transfected with the lyophilized products of Formulations 7 and 9.

### DETAILED DESCRIPTION OF THE INVENTION

The present disclosure is further described below in conjunction with the examples, but these examples are not intended to limit the scope of the present disclosure.

The experimental methods without specifying specific conditions in the examples of the present disclosure were generally conducted under conventional conditions, or conditions recommended by the manufacturers of the starting materials or commercial products. The reagents without specifying specific sources were conventional reagents purchased from the market.

### Example 1:

### Preparation of lipid nanoparticles

Compound 1 (the compound of formula I, prepared with reference to WO2023125738A), DSPC, cholesterol, and a DMG-PEG 2000 solution were mixed at certain molar percentages (mol%) (i.e., the percentage of the molar amount of each component to the molar amount of total lipids present in the lipid nanoparticles) to prepare an ethanol lipid solution. The four components were used for the preparation according to the molar ratios shown in Table 1. mRNA encoding luciferase protein was dissolved in a acetate buffer (pH 4) to prepare an aqueous mRNA solution. The ethanol lipid solution and the aqueous mRNA solution were mixed by microfluidics at a volume ratio of 1:3 (the weight ratio of the total lipids to the mRNA being approximately 20:1) to prepare liposomes. The ethanol was removed by dialysis against a 20 mM Tris solution, to obtain a liposomal nanoparticle composition in which the mRNA was encapsulated.

### Characterization of the lipid nanoparticle composition

### Characterization method

The particle size and polydispersity index PDI of the liposomal nanoparticles were measured using a Malvern Zetasizer Pro in a 173° backscatter detection mode with dynamic light scattering.

The liposome encapsulation efficiency was determined using a Quant-iT RiboGreen RNA Assay Kit for the quantitative detection of RNA.

**Table 1**

| No. | Compound 1 mol% | DSPC mol% | Cholesterol mol% | PEG-D MG mol% | Particle size nm | PDI | Encapsulation efficiency % |
|---|---|---|---|---|---|---|---|
| 1 | 48.0 | 10.0 | 40.5 | 1.5 | 90.8 | 0.12 | 97 |

### Example 2:

### Evaluation of lyophilization of the lipid nanoparticle composition

Lipid nanoparticles were prepared according to the lipid composition in Example 1, and the prepared lipid nanoparticles were exchanged into buffer solutions (containing 20 mM Tris (pH 7.5), 8% sucrose, 5% trehalose, and different concentrations of sodium chloride solutions corresponding to those in the table below) for lyophilized formulations by ultrafiltration. The lyophilized formulation solutions of lipid nanoparticles were filled into 2-mL vials, 0.6 mL/vial, with a cationic lipid concentration of 1.78 mg/mL in each vial. The vials were pre-frozen at -45°C for 3 hours, then dried firstly at -40°C for 30 hours, and dried secondly at 30°C for 10 hours under a vacuum of 0.05 mmbar. The resulting product was the final lyophilized product.

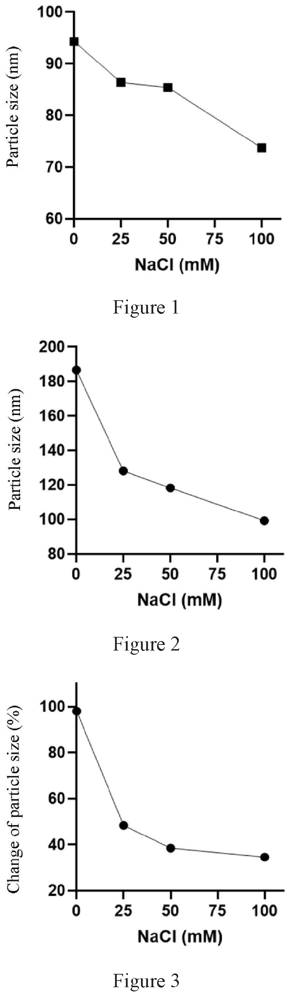

The lyophilized products were reconstituted by adding 0.6 mL of water for injection, and the particle size of lipid nanoparticles was measured according to the aforementioned method. The particle sizes of lipid nanoparticles before and after lyophilization were compared. The measurement results are shown in Table 2 and Figures 1-3. The sodium chloride in the formulations significantly reduced the particle sizes of lipid nanoparticles before and after lyophilization, and also significantly reduced the rate of particle size change after lyophilization and reconstitution.

**Table 2**

| No. | NaCl concentration (mM) | Mass ratio of sodium chloride to the cationic lipid | Particle size before lyophilization (nm) | Particle size after lyophilization and reconstitution (nm) | Change rate (%) |
|---|---|---|---|---|---|
| Formulation 1 | 0 | 0 | 94.3 | 186.7 | 98.0 |
| Formulation 2 | 25 | 1:1.2 | 86.4 | 128.2 | 48.4 |
| Formulation 3 | 50 | 1.6:1 | 85.4 | 118.3 | 38.5 |
| Formulation 4 | 100 | 3.3:1 | 73.7 | 99.2 | 34.6 |

The lipid nanoparticle stock solutions in the experiment above were diluted to one-half and one-third concentrations using the buffer solutions for lyophilized formulations, and the prepared lipid nanoparticles were filled and lyophilized according to the above experimental procedures. The particle size of lipid nanoparticles after lyophilization and reconstitution was measured. The measurement results are shown in Table 3.

**Table 3**

| No. | NaCl concentration (mM) | Mass ratio of sodium chloride to the cationic lipid | Particle size before lyophilization (nm) | Particle size after lyophilization and reconstitution (nm) | Change rate (%) |
|---|---|---|---|---|---|
| Formulation 5 | 100 | 6.6:1 | 74.7 | 101.4 | 35.7 |
| Formulation 6 | 100 | 9.9:1 | 73.4 | 100.4 | 36.8 |

The prepared lipid nanoparticles were exchanged into another group of buffer solutions (containing 20 mM Tris (pH 7.5), 8% sucrose, and different concentrations of sodium chloride solutions corresponding to those in the table below) for lyophilized formulations by ultrafiltration for buffer exchange. The lyophilized formulation solutions of lipid nanoparticles were filled into 2-mL vials at a volume of 0.6 mL/vial, with a cationic lipid concentration of 2.38 mg/mL in each vial. The vials were placed in a lyophilizer. The vials were pre-frozen at -45°C for 3 hours, then were dried firstly at -40°C for 30 hours, and dried secondly at 30°C for 10 hours under a vacuum degree of 0.05 mmbar. The resulting product was the final lyophilized product.

The lyophilized products were reconstituted by adding 0.6 mL of water for injection, and the particle size of lipid nanoparticles was measured according to the aforementioned method. The particle sizes of lipid nanoparticles before and after lyophilization were compared. The measurement results are shown in Table 4.

**Table 4**

| No. | NaCl concentration (mM) | Mass ratio of sodium chloride to the cationic lipid | Particle size before lyophilization (nm) | Particle size after lyophilization and reconstitution (nm) | Change rate (%) |
|---|---|---|---|---|---|
| Formulation 7 | 0 | 0 | 93.5 | 162.6 | 73.9 |
| Formulation 8 | 25 | 1:1.6 | 81.5 | 121.8 | 49.4 |
| Formulation 9 | 50 | 1.2:1 | 82.0 | 121.7 | 48.4 |

### Example 3:

### Evaluation of integrity of mRNA in the lipid nanoparticle composition

Lyophilized lipid nanoparticles were obtained according to Example 2, and the integrity of mRNA in each group of samples was detected using a capillary electrophoresis instrument (5200 fragment analyzer from Agilent).

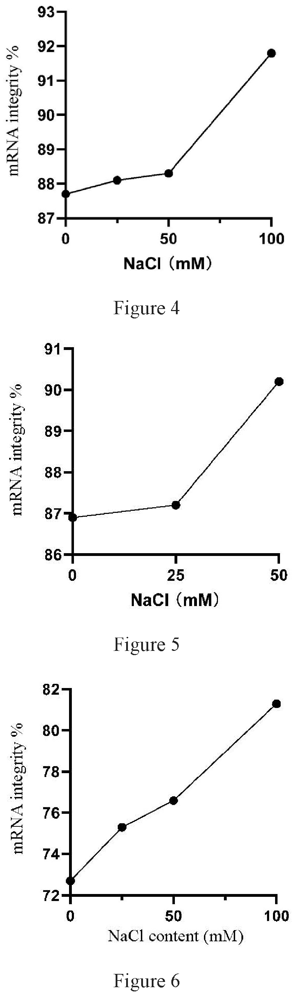

mRNA is easily degraded, and may be affected by temperature. As the temperature increases, the stability of mRNA may decrease. With prolonged storage time, degradation occurs under high temperature conditions, leading to decrease in integrity. During the freeze-drying process, there is a temperature increase from the first drying to the second drying stage, with the second drying temperature being higher. Therefore, the mRNA integrity was evaluated for different formulations with sodium chloride during the freeze-drying process and under high-temperature storage conditions. As shown in Tables 5-6 and Figures 4-7, the mRNA integrity after removal from the lyophilization chamber was enhanced with increasing the sodium chloride concentration. Likewise, under the high temperature storage condition at 37°C, the mRNA integrity was enhanced with increasing the sodium chloride concentration.

**Table 5**

| No. | NaCl concentration (mM) | MRNA integrity (%) | |
|---|---|---|---|
| | | After removal from the lyophilization chamber | At 37°C for 72 hours |
| Formulation 1 | 0 | 87.7 | 72.7 |
| Formulation 2 | 25 | 88.1 | 75.3 |
| Formulation 3 | 50 | 88.3 | 76.6 |
| Formulation 4 | 100 | 91.8 | 81.3 |

**Table 6**

| No. | NaCl concentration (mM) | MRNA integrity (%) | |
|---|---|---|---|
| | | After removal from the lyophilization chamber | At 37°C for 72 hours |
| Formulation 7 | 0 | 86.9 | 72.9 |
| Formulation 8 | 25 | 87.2 | 77.2 |
| Formulation 9 | 50 | 90.2 | 80.0 |

### Example 4:

### Evaluation of mRNA delivery efficiency of the lipid nanoparticles on cells

HEK 293 cells were seeded into a 96-well plate and incubated overnight. When the cell density reached 80% or more, the lipid nanoparticle solution in which the luciferase mRNA was encapsulated (the lyophilized product of Formulation 7 and the lyophilized product of Formulation 9) was added to the culture medium in the cell plate wells at mRNA dose of 100 ng/well. After 24 hours, the fluorescence intensity of the expressed luciferase protein was measured using a luciferase reporter gene assay kit (Promega) and a microplate reader. The fluorescence intensity values, i.e., the fluorescence measurements detected by the microplate reader, represent expression levels of luciferase protein. The higher the fluorescence intensity, the higher the protein expression level. The mean fluorescence intensity was calculated for at least three replicates for each lipid nanoparticle compound

**Table 7**

| No. | Mean fluorescence intensity |
|---|---|
| Formulation 7 | 2.34E+07 |
| Formulation 9 | 4.12E+07 |

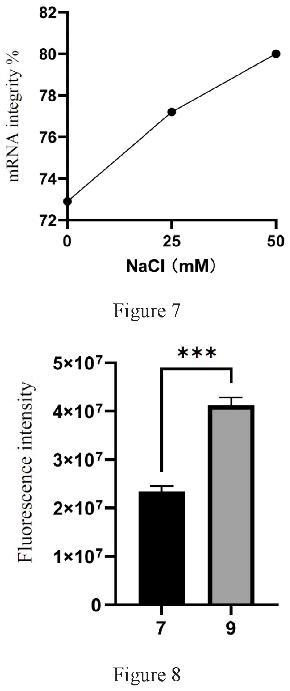

Conclusion: as shown in Table 7 and Figure 8, the fluorescence intensity indicated that the expression level of the lyophilized lipid nanoparticles corresponding to Formulation 9 was significantly higher than that of Formulation 7. *** in Figure 8 represents a statistically significant difference, P<0.001.

### Example 5:

### Preparation process of HGF-mRNA-LNP spray formulation:

HGF-mRNA-LNPs (0.09 mg of HGF mRNA, 1.07 mg of cationic lipid of formula I; the percentages of the molar amounts of the cationic lipid, DSPC, cholesterol and DMG-PEG 2000 to the total molar amount of lipid nanoparticles were 48 mol%, 10 mol%, 40.5 mol%, and 1.5 mol%, respectively) were prepared according to the method in Example 1. HGF-mRNA-LNP lyophilized powder (Formulation 10) was prepared according to Formulation 4 in Example 2. The lyophilized product was reconstituted by adding 0.6 mL of water for injection, and the particle size of lipid nanoparticles was measured according to the aforementioned method. The particle sizes of lipid nanoparticles before and after lyophilization were compared.

**Table 8**

| No. | NaCl concentration (mM) | Mass ratio of sodium chloride to the cationic lipid | Particle size before lyophilization (nm) | Particle size after lyophilization and reconstitution (nm) | Change rate (%) |
|---|---|---|---|---|---|
| Formulation 10 | 100 | 3.3:1 | 76.8 | 102.5 | 33.5 |

### Example 6:

### Preparation process of anti-PD-1 antibody-mRNA-LNP spray formulation:

Anti-PD-1 antibody-mRNA-LNPs were prepared according to the method in Example 1. Anti-PD-1 antibody-mRNA-LNP lyophilized powder was prepared according to Formulation 4 in Example 2. The lyophilized product was reconstituted by adding 0.6 mL of water for injection, and the particle size of lipid nanoparticles was measured according to the aforementioned method. The particle sizes of lipid nanoparticles before and after lyophilization were compared.

## Claims

1. A lyophilized composition, comprising lipid nanoparticles and sodium chloride, wherein the lipid nanoparticles comprise at least one cationic lipid, and the weight ratio of sodium chloride to the cationic lipid ranges from 1:10 to 10:1; preferably, the weight ratio of sodium chloride to the cationic lipid ranges from 1:5 to 10:1; more preferably, the weight ratio of sodium chloride to the cationic lipid ranges from 1:5 to 5:1; more preferably, the weight ratio of sodium chloride to the cationic lipid ranges from 1:3 to 5:1; more preferably, the weight ratio of sodium chloride to the cationic lipid ranges from 1:2 to 5:1; and more preferably, the weight ratio of sodium chloride to the cationic lipid ranges from 1:2 to 4:1.

2. The lyophilized composition according to claim 1, wherein the cationic lipid is a compound of formula A, 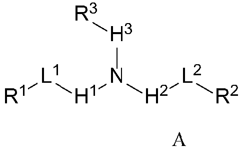
wherein, L¹ and L² are each independently selected from the group consisting of -C(O)O-, and -OC(O)-;
H¹ and H² are each independently selected from the group consisting of C₁₋₁₂ heteroalkylene, C₁₋₁₂ alkylene, and C₂₋₁₂ alkenylene;
H³ is selected from the group consisting of C₁₋₂₄ alkylene, C₁₋₂₄ heteroalkylene, and C₂₋₂₄ alkenylene;
R¹ and R² are each independently selected from the group consisting of C₁₋₂₄ alkyl and C₂₋₂₄ alkenyl;
R³ is -OR⁵, and R⁵ is hydrogen.

3. The lyophilized composition according to claim 1, wherein the cationic lipid is selected from the group consisting of N,N-dioleyl-N,N-dimethylammonium chloride (DODAC), N,N-distearyl-N,N-dimethylammonium bromide (DDAB), 1,2-dioleoyltrimethylammonium propane chloride (DOTAP)(also known as N-(2,3-dioleoyloxy)propyl)-N,N,N-trimethylammonium chloride and 1,2-dioleoyloxy-3-trimethylaminopropane chloride salt), N-(1-(2,3-dioleoyloxy)propyl)-N,N,N-trimethylammonium chloride (DOTMA), N,N-dimethyl-2,3-dioleoyloxy)propylamine (DODMA), 1,2-dilinoleyloxy-N,N-dimethylaminopropane (DLinDMA), 1,2-dilinolenyloxy-N,N-dimethylaminopropane (DLenDMA), 1,2-di-γ-linolenyloxy-N,N- dimethylaminopropane (γ-DLenDMA), 1,2-dilinoleylcarbamoyloxy-3-dimethylaminopropane (DLin-C-DAP), 1,2-dilinoleyloxy-3-(dimethylamino)acetoxypropane (DLin-DAC), 1,2-dilinoleyloxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazinyl)propane (DLin-MPZ) or 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cy clopenta[d][1,3]dioxol-5-amine, (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl) piperazin-1-yl)ethylazanediyl)didodecan-2-ol (C12-200), 2,2-dilinoleyl-4-(2-dimethylaminoethyl)-[1,3]-dioxolane (DLin-K-C2-DMA), 2,2-dilinoleyl-4-dimethylaminoethyl-[1,3]-dioxolane (DLin-K-DMA), (6Z,9Z,28Z,31Z)-heptatriaconta-6,9,28,31-tetraen-19-yl 4-(dimethylamino)butanoate (DLin-M-C3-DMA), 3-((6Z,9Z,28Z,31Z)-heptatriaconta-6,9,28,31-tetraen-19-yloxy)-N,N-dimethylpropan-1-amine (MC3 Ether), 4-((6Z,9Z,28Z,31Z)-heptatriaconta-6,9,28,31-tetraen-19-yloxy)-N,N-dimethylbutan-1-amine (MC4 Ether), ((4-hydroxybutyl)azanediyl)bis(hexan-6,1-diyl)bis(2-hexyldecanoate) (ALC-0315), 8-[(2-hydroxyethyl)[6-oxo-6-(undecyloxy)hexyl]amino]octanoic acid 1-octylnonyl ester heptadecan-9-yl-8-((2-hydroxyethyl)(6-oxo-6-((decyloxy)hexyl)amino)octoate)(SM-1 02), a compound of formula I, or any combination of the above-mentioned cationic lipids,

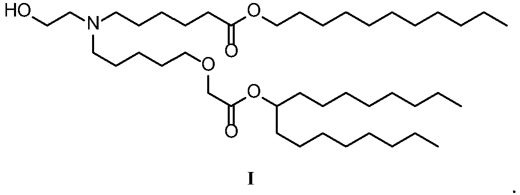

4. The lyophilized composition according to any one of claims 1 to 3, wherein the cationic lipid comprises from 20 mol % to 70 mol % of the total lipids present in the lipid nanoparticles; preferably, the cationic lipid comprises from 30 mol % to 70 mol % of the total lipids present in the lipid nanoparticles; and more preferably, the cationic lipid comprises from 40 mol % to 70 mol % of the total lipids present in the lipid nanoparticles.

5. The lyophilized composition according to any one of claims 1 to 4, wherein the lipid nanoparticles comprise at least one non-cationic lipid, preferably the non-cationic lipid is selected from a mixture of a phospholipid and cholesterol or its derivative thereof.

6. The lyophilized composition according to claim 5, wherein the non-cationic lipid comprises from 20 mol % to 70 mol % of the total lipids present in the lipid nanoparticles; preferably, the non-cationic lipid comprises from 30 mol % to 60 mol % of the total lipids present in the lipid nanoparticles; and more preferably, the non-cationic lipid comprises from 20 mol % to 50 mol % of the total lipids present in the lipid nanoparticles.

7. The lyophilized composition according to claim 5, wherein the phospholipid comprises from 5 mol % to 30 mol % of the total lipids present in the lipid nanoparticles.

8. The lyophilized composition according to claim 5, wherein cholesterol or its derivative thereof comprises from 20 mol % to 60 mol % of the total lipids present in the lipid nanoparticles; preferably, cholesterol or its derivative thereof comprises from 30 mol % to 60 mol % of the total lipids present in the lipid nanoparticles; and more preferably, cholesterol or its derivative thereof comprises from 20 mol % to 45 mol % of the total lipids present in the lipid nanoparticles.

9. The lyophilized composition according to claim 1, wherein the lipid nanoparticles further comprise at least one conjugated lipid; preferably, the conjugated lipid in the lyophilized composition comprises from 0.5 mol % to 5 mol % of the total lipids present in the lipid nanoparticles.

10. The lyophilized composition according to claim 1, wherein the lyophilized composition does not comprise poloxamer or potassium sorbate.

11. The lyophilized composition according to claim 1, wherein the lipid nanoparticles further comprise an active agent selected from nucleic acids, preferably the nucleic acid is selected from the group consisting of ribonucleic acids and deoxyribonucleic acids, and more preferably selected from the group consisting of any one or any combination of small interfering RNA (siRNA), microRNA (miRNA), small hairpin RNA (shRNA), and messenger RNA (mRNA).

12. The lyophilized composition according to any one of claims 1 to 11, comprising
a) sodium chloride, the weight ratio of sodium chloride to the cationic lipid ranging from 1:5 to 10:1;
b) lipid nanoparticles comprising:
an active agent, wherein the active agent includes mRNA;
a cationic lipid comprising a compound of formula I or a pharmaceutically acceptable salt thereof, wherein the cationic lipid comprises from 10 mol % to 75 mol % of the total lipids present in the lipid nanoparticles, 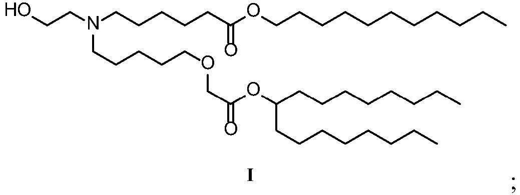
a non-cationic lipid selected from the group consisting of a phospholipid and cholesterol or a derivative thereof, wherein the phospholipid comprises from 5 mol % to 40 mol % of the total lipids present in the lipid nanoparticles, and the cholesterol or the derivative thereof comprises from 15 mol % to 60 mol % of the total lipids present in the lipid nanoparticles; and
a conjugated lipid comprise from 0.5 mol % to 2 mol % of the total lipids present in the lipid nanoparticles.

13. The lyophilized composition according to any one of claims 1 to 12, further comprising a lyoprotectant; preferably, the lyoprotectant is selected from one or more of sucrose, trehalose, mannitol, and maltose; preferably, the lyoprotectant is selected from sucrose, and a combination of sucrose and trehalose.

14. The lyophilized composition according to claim 13, comprising a lyoprotectant at a concentration from 5% w/v to 20% w/v, and preferably at a concentration from 5% w/v to 15% w/v.

15. The lyophilized composition according to any one of claims 1 to 14, comprising from 5% w/v to 15% w/v sucrose, from 5% w/v to 10% w/v sucrose or from 1% w/v to 10% w/v trehalose.

16. The lyophilized composition according to any one of claims 11 to 15, wherein the mRNA comprises an open reading frame (ORF), and the ORF encodes a protein or a polypeptide;
preferably, the mRNA further comprises any one or any combination of a 5' untranslated region element (5'UTR), a 3' untranslated region element (3'UTR), and a polyadenylic acid (poly-A) tail;
more preferably, the mRNA further comprises a 5'Cap structure (5'Cap).

17. The lyophilized composition according to claim 16, wherein the protein or polypeptide is an antigen,
preferably, the antigen is selected from viral antigen, bacterial antigen, and cancer antigen;
more preferably, the viral strain is selected frominfluenza virus, coronavirus, varicella zoster virus (VZV), and respiratory syncytial virus (RSV).

18. The lyophilized composition according to claim 16, wherein the protein or polypeptide is hepatocyte growth factor (HGF), antibody or antigen-binding fragment thereof.

19. The lyophilized composition according to any one of claims 1 to 18, wherein, after reconstitution of the lyophilized composition, the average particle size of the lipid nanoparticles increases by no more than 100%; preferably, the average particle size of the lipid nanoparticles increases by no more than 80%; and preferably, the average particle size of the lipid nanoparticles increases by no more than 60%.

20. The lyophilized composition according to any one of claims 1 to 19, wherein the lyophilized composition is tested for mRNA integrity immediately after being removed from the lyophilization chamber, wherein the mRNA integrity is not less than 75%, or not less than 80%, or not less than 85%, or not less than 86%, or not less than 87%, or not less than 88%, or not less than 89%, or not less than 90%.

21. The lyophilized composition according to any one of claims 1 to 20, wherein the lyophilized composition is tested for mRNA integrity after being placed at 37°C for 72 h, wherein the mRNA integrity is not less than 75%, or not less than 80%, or not less than 85%, or not less than 86%, or not less than 87%, or not less than 88%, or not less than 89%, or not less than 90%.

22. A method for preparing a lyophilized composition, comprising the following steps:
a. providing a suspension of the lipid nanoparticles as defined in claims 1 to 21 in a liquid medium;
b. adjusting the liquid medium to form a suspension containing NaCl; and
c. lyophilizing;
wherein the liquid medium contains 10-200 mM NaCl, preferably 10-150 mM NaCl, more preferably 20-150 mM NaCl, more preferably 20-120 mM NaCl, more preferably 50-150 mM NaCl, more preferably 50-120 mM NaCl, and more preferably 70-120 mM NaCl.

23. A reconstituted solution obtained by a step of reconstituting the lyophilized composition according to claims 1 to 21 or a lyophilized composition prepared by the preparation method according to claim 22, wherein the solvent used for the reconstitution is preferably water for injection.

24. Use of the lyophilized composition according to any one of claims 1 to 21 or the reconstituted solution according to claim 23 in the preparation of a medicament for inducing a protective immune response in a mammal, preferably in the preparation of a vaccine.

25. Use of the lyophilized composition according to any one of claims 1 to 21 or the reconstituted solution according to claim 23 in the preparation of a medicament for treating a disease or dysfunction in a mammal, wherein the disease is preferably selected from viral infection, liver disease, cancer, ischemic disease, metabolic syndrome, diabetes and its complication.
